# EUROPEAN PATENT APPLICATION

(11) **EP 2 017 613 A1**
(43) Date of publication of application: **21.01.2009**
(21) Application number: 08160682.4
(22) Date of filing: 18.07.2008
(51) Int. Cl.: G01N 29/02, G01N 29/036, G01N 29/22, G01N 29/30

(54) **Piezoelectric biosensor and biosensor array for parallel detection of multiple biomarkers**

(30) Priority: 20.07.2007 EP 07112863
(71) Applicant: Genetel Pharmaceuticals Limited, Hong Kong (HK)
(72) Inventor: Yang, Mengsu Unit B, 8th Floor, Senior Staff Quarters, 81 Tat Chee Avenue Kowloon Shandong (CN); Cheung, Pik Yuen, Tokwawan Kowloon Hong Kong (CN); Tzang, Chi Hung, Tokwawan Kowloon Hong Kong (CN); Su, Li, Jinan Shandong (CN)
(74) Representative: Ziebig, Marlene

(57) **Abstract**

Biosensors are analytical devices composed of a recognition element coupled to a physical transducer (mass, optical, electrochemical and thermal) for qualitative and / or quantitative detection of biological analytes. Biosensors have wide applications in Medical & Technology; Food & Technology; Industrial & Technology; Environment Measurement & Technology and BioDefense.

The invention is about a novel biosensor and biosensor array using piezoelectric ceramic resonators as the core component of the biosensor, and to develop a biosensor platform for simultaneous detection of multiple biomarkers including but not limited to detection of multiple cancer markers for screening and early diagnosis of the most common types of cancers; detection of multiple heart disease markers for early diagnosis and prognosis of heart disease; detection of multiple food contaminants for improvement of food safety; detection of multiple environmental toxins for monitoring environmental pollution and detection of multiple bio-warfare agents for military and civil defence applications. The piezoelectric ceramic-based biosensor and the technology platform established will create an exciting new market in various sectors with tremendous growth potential.

## Description

### FIELD OF THE INVENTION

The invention relates to a high-frequency ceramic piezoelectric biosensor and biosensor array for the parallel detection and analysis of biomarkers in various samples by detecting the change in frequency before and after sample introduction using either a dry condition or a wet condition process. In particular, the technology platform consists of a piezoelectric ceramic biosensor with capture molecules for biomarkers, a sample loading cartridge, a computation system for signal output and analysis for monitoring biomarker levels in samples such as blood, serum, plasma, urine etc. The biosensor array can be used for simultaneous detection of multiple biomarkers including but not limited to detection of multiple cancer markers for screening and early diagnosis of the most common types of cancers; detection of multiple heart disease markers for early diagnosis and prognosis of heart disease; detection of multiple food contaminants for improvement of food safety; detection of multiple environmental toxins for monitoring environmental pollution and detection of multiple bio-warfare agents for military and civil defence applications.

### BACKGROUND OF THE INVENTION

### Biosensor technology

Biosensors are analytical devices composed of a recognition element coupled to a physical transducer measuring mass, optical, electrochemical or thermal properties for qualitative and quantitative detection of biological analytes.

Biosensors have wide applications in the following areas: medical & technology applications; food & technology applications; industrial & technology applications; environment measurement & technology applications; and biodefense applications. The increasing trend in healthcare strives for early detection for disease screening and prevention. The increasing rate of obesity and the alarming rise in the rate of diabetes in industrialized world is driving a need for biosensors to monitor diabetic patients' glucose levels. The increasing pollution and food safety problems are driving the development of biosensors for food and environmental applications. The pharmaceutical industry is driving the need for new and rapid biosensors to speed up drug discovery rate. The war-on-terrorism is driving the need for new and rapid detection biosensors against bio-warfare agents for military and civil defence applications. While the demand for biosensors in each of the above five areas increases all at a high annual rate, the application in the medical area overshadows the other seemingly important application areas.

### Piezoelectric quartz crystals

Piezoelectric quartz crystal has been developed as biosensors for the detection of DNA, protein, virus, bacteria, living cells toxin as described in Marx, K.A. Quartz crystal microbalance: a useful tool for studying thin polymer films and complex biomolecular systems at the solution-surface interface, Biomacromolecules, Vol 4(5), 1099-1120, 2003, [1]; and applied in biochemical, environmental, food and clinical analyses for various hydrocarbons [2], gas-phase analytes [3], pollutants [4, 5]. Numerous combinations of piezoelectric device with immunological elements have been reported in Yuan, J.-B., Tan, Y.-G., Nie, L.-H., Yao, S.-Z., 2002. Piezoelectric quartz crystal sensors based on ion-pair complexes for the determination of cinchonine in human serum and urine. Anal. Chim. Acta 454, 65-74; and Su, X.-D., Chew, F.-T., Li, S.F.Y., 2000. Piezoelectric quartz crystal based label-free analysis for allergy disease. Biosens. Bioelectr. 15, 629-639 [6, 7]. Nevertheless, as the sensitivity of piezoelectric biosensors depends on the oscillating frequency, the area and thickness of the material, there are technical difficulties in producing thinner, smaller quartz with higher frequency. These physical barriers limit the application of quartz crystal biosensors due to their low sensitivity and high cost.

Moreover, it is noted that in an article entitled "Quartz crystal microbalance: a useful tool for studying thin polymer films and complex biomolecular systems at the solution-surface interface," Marx, K.A., Biomacromolecules, Vol 4(5), 1099-1120, 2003, an electromechanical quartz crystal microbalance QCM is described [1]. However, quartz microbalances have the sensitivity problems noted above, and as compared with the subject ceramic-based product, the differences are as follows:

| Parameters | Ceramic | Quartz |
|---|---|---|
| Sensing material | Ceramic resonator | Quartz crystal |
| Frequency | 40-100MHz | 10MHz |
| Sample for detection | Protein/DNA/carbohydrate/ different biological markers etc. | Not specified |
| Markers to be screened | Multiple | Multiple for certain model no. |
| Max number of markers to be detected simultaneously | 16 | 4 to 8 |
| Measuring condition | Protein-protein interaction/DNA hybridization/ligand receptor etc. | Electrochemical reaction |
| | Dry and wet | Liquid |
| | Single measurement | Not specified |
| Measurement | Resonance frequency/impedance/ resistance/capacitance change | Potentiostats / Galvanostats |
| Sample volume required | Little (few µl) | Large (several ml) |
| Sample detection range | ng-µg | ng-mg |
| Detector size | Small | Large |
| Sensor | Disposable | Reuse |

| | | |
|---|---|---|
| Note that the quartz device measures electrochemical change of the sample. In this system, the quartz crystal is sealed to the side opening in a measuring cell wall. Cell volumes of 3.5, 5, 10, 25, 50, and 100 mL are available. In one embodiment of the quartz device, the measurement is under liquid condition that depends on an acoustic wave measurement rather than a resonance frequency change measurement as will be described for the subject system. | | |

WO 91/05261 "Assay method for biological target complexes on the surface of a biosensor" [8] is also included here as reference which described the use of A/T cut thickness shear mode quartz crystal as the sensing material for sample detection. The detection is depending on the acoustic wave propagation. However, due to the sensitivity problems noted above, it used complicated steps for analyzing the presence of target in sample through indirect measurement of mass changes. The capturing molecule is immobilized on the sensor surface which is a general capturing molecule e.g. avidin for capturing different molecules containing biotin. The biotin containing molecule (the bifunctional conjugate) could be specific antibodies to different analytes. The bifunctional conjugate would bind to the analyte and be captured by the capturing molecule on the sensor surface. To detect the signal, a substrate that would lead to color change due to the interaction between the capturing molecule and the bifunctional conjugate would be added that would be deposited on the sensor surface and lead to frequency change. This setup is being used due to the low sensitivity of the setup for direct mass detection that would require amplification using signal generating product deposit on the sensor surface. Besides, the complicated procedures described by WO 91/05261 may not be directly applicable in the clinical diagnostic purpose as simplicity, speed, high through put and low cost are desirable.

On the other hand, piezoelectric ceramics offer the advantage of high ruggedness and ease of fabrication in complex shapes and flexibility due to the higher frequencies at which they can operate and due to their reduced cost. However, the application of ceramics to biological sample detection is limited.

### Piezoelectric ceramic-based biosensor

Note that Piezoelectricity is a phenomenon exhibited by noncentrosymmetric crystals whereby an electric polarization or charge is induced in the material upon the application of a stress. Piezoelectricity exists in some naturally occurring crystals such as quartz, but the bulk of the piezoelectric materials come from synthetic polycrystalline ferroelectric ceramics, such as lead titanate zirconate (PZT). The coupling of electrical and mechanical energy makes piezoelectric materials useful in a wide range of applications including sensors and actuators, telecommunication, and energy conversion such as described in Setter N ed., Piezoelectric materials in devices, Ceramics Laboratory, EPFL, Switzerland, 2002. [9].

For the biosensors using ceramic materials, most of the applications describe systems that utilize the ceramic material as an underlying material for coating another sensing material. Moreover, the measurement depends on electrochemical changes rather than utilizing the ceramic material's piezoelectric property that enables direct frequency measurement.

For ceramic biosensor application, the cantilever [10, 11] or the acoustic property [12, 13] of the material is utilized rather than the direct measurement of resonance frequency change for sample measurement. See Atonomics APS, Microsensors and method for detecting targeting analytes, WO 2002/020832, 14.03.2002; Drexel University, Piezoelectric cantilever sensor, WO 2005/043126, 12.05.2005; Valtion Teknillinen Tutkimuskeskus, Micromechanical sensor, sensor array and method, WO 2007/006843, 18.01.2007 and Atonomics APS, Surface acoustic wave sensor comprising a hydrogel, EP 1804059, 04.07.2007.

More than 150 publication related to piezoelectric biosensor have been published, while only a few publications are related to the utilization of ceramic piezoelectric property in biosensor applications, namely in Lee JH, Hwang KS, Park J, Yoon KH, Yoon DS, Kim TS. Immunoassay of prostate-specific antigen (PSA) using resonant frequency shift of piezoelectric nanomechanical microcantilever. Biosens Bioelectron. 2005 Apr 15;20(10):2157-62; Verissimo MIS, Mantas PQ, Senos AMR, Oliveir JABP, Gomes MTSR. Suitability of PZT ceramics for mass sensors versus widespread used quartz crystals. Sensors and Actuators B 2003; 95:25-31; and Eklund A, Backlund T, Lindahl OA. A resonator sensor for measurement of intraocular pressure--evaluation in an in vitro pig-eye model. Physiol Meas. 2000 Aug;21 (3):355-67 [14, 15, 16].

For instance, with respect to EP 1 804 059, acoustic wave sensors detect materials by generating and observing an acoustic wave was described. The detection is based on acoustic wave propagates through or on the surface of the material (in this case by surface acoustic wave) caused changes to the characteristics of the propagation path on velocity and/or amplitude of the wave. The SAW sensor design has interidigital gold electrodes constructed on a piezoelectric supporting comprising lithium tantalite and each sensor was coated with SiO₂ for electric insulation and chemical attachments purpose. The recognizing element is embedded in the hydrogel which are attached to the sensor surface after polymerization process. The amplitude, frequency, and/or phase characteristics of the sensor were measured and correlated to a corresponding physical quantity due to analyte application. Application of sample containing analyte leads to change of both mass and property of hydrogel that causes the frequency change.

As to WO 02/20832, the use of microscopic flexible mechanical structures such as micro-cantilevers or micromembranes as non-fluorescent sensor. Binding of the analyte induce stress of the microsensor which result in deflection of the microsensor. The deflection is detected by change in electrical parameter of piezoelectric element e.g. voltage, resistance and current. The detection is based on the following principle: as the cantilever scans across the indentation of a polymer film, the cantilever deflects along the indentation of the film, causing stress on the PZT film. The variation of stress in the PZT film produces self-generated charges on the surface of the PZT capacitor without applying voltage. The charges are not generated by the absolute stress, but by the variation of stress in which stress leads to frequency change. Variations in stress measurements are, however different in sensing mechanism to direct absolute mass resonance sensing, which is different from the system described herein.

With respect to the Verissimo reference, according to the information in this article, the preparation of PZT used in this article is (Nb-Mn-PZT). The ceramic disc has a relatively low frequency range of 100-200kHz, and it has been found that the viability of such a low-frequency system for use as a biosensor is questionable.

As to the Eklund et al. system, rod shape PZT with dimension of 25x5x1 mm was used as the sensor element for measuring pressure change through frequency shift between unloaded and loaded conditions. The unloaded resonance frequency was approximately 82.9kHz and the measuring range is 200-700Hz. The PZT used are with a much larger size and detection is in the low frequency range and not comparable the ceramic resonators described herein and not suitable for development into array type sensor for multiple sample measurement.

Finally, with respect to the utilization of PZT, Jong Hun Lee et al., in an article entitled "Immunoassay of prostate-specific antigen (PSA) using resonant frequency shift of piezoelectric nanomechanical microcantilever" Biosens Bioelectron, 2005 Apr 15;20(10:2157-62, describes a PCT cantilever device that measures resonant frequency change. The Jong Hun Lee et al. article basically describes a stress measurement of frequency change. In a piezoelectric cantilever system such as the PZT cantilever device of Jong Hung Lee et al., the sensing mechanism is described above. The nanomechanical PZT cantilevers are homemade with resonance frequency of 16kHz or 61kHz depending on the size of the PZT used in the experiment and the measurement range is from 100-400Hz.

If a biosensor platform could be developed using a piezoelectric ceramic resonator and resonant frequency change as the core component of detection, the platform could be applied to detection of various biomarkers in different fields of application. As clinical applications have the greatest share in the biosensor market and because cancer is the leading cause of death worldwide, biosensors for detection of multiple cancer markers address many market needs.

### Cancer is a global healthcare problem

Cancer is the uncontrolled growth and spread of cells that may affect almost any tissue of the body. More than 11 million people are diagnosed with cancer every year. It is estimated that there will be 16 million new cases every year by 2020. Cancer causes 7.6 million deaths every year-or 13% of deaths worldwide [17]. Cancer is now the leading cause of death in China and Hong Kong whereas malignant neoplasms lead to a death rate of 23.92% in China in 2004 [18] and 31.8% in HK in 2005 [19] respectively.

Lung, colorectal and stomach cancer are among the five most common cancers in the world for both men and women. Among men, lung and stomach cancer are the most common cancers worldwide. For women, the most common cancers are breast and cervical cancer [20, 21]. In China, approximately 1.3 million people die of cancer each year. The main cancer sites are stomach, liver, lung, and oesophagus. These four cancers account for 74% of all cancer deaths in the country. Among all deaths from cancer, lung cancer is the most common, accounting for 22.0% of all cancer deaths for males, followed by stomach cancer with 17.8% and hepatic cancer with 13.0% while stomach cancer is the most common for females with 14.8%, followed by lung cancer with 12.7% and colon cancer with 10.1 %. Stomach cancer and cervix/corpus uteri cancer which used to be major causes of death among the Japanese now show decreasing trends, indicating the advance of medical technologies such as early detection and early treatment is also a relevant factor.

Cancer is largely preventable: around one-third of all cancers are preventable by stopping smoking, providing healthy food and avoiding the exposure to carcinogens. Some of the most frequent cancer types are curable by surgery, chemotherapy or radiotherapy if early detection is available.

### Cancer diagnostic methods

Throughout the years, researchers are working hard in developing various methods for diagnosis of cancer. Most of the cancer diagnostic assays depend on biomarker detection for diagnosis, staging, prognosis and management of patients with cancer. Biomarkers are substances that can often be detected in higher-than-normal amounts in the blood, urine, or body tissues of some patients with certain types of cancer. Cancer markers are produced either by the tumour itself or by the body in response to the presence of cancer or certain benign (noncancerous) conditions.

Immunoassays (direct detection of cancer protein markers) and histology (detection of cancerous cells through cancer protein markers) currently command the majority of the cancer diagnostics market. Most of the immunoassays only target a single cancer marker.

### Need for development of new cancer diagnostic technology

Cancer diagnostics are a naturally growing market, owing to the increased incidence of cancer and an aging population. An aging population will lead to increased number of cancer patients. Unhealthy lifestyles are the other causes for increased cancer incidence, such as smoking, poor or imbalanced diet, inactive lifestyles, and increased exposure to ultraviolet light and polluted environments.

Most diagnostic assays are based on immunoassays (such as ELISA) which are widely available and are routinely employed in the clinical laboratory. Normally a single marker is detected by each ELISA kit. For multiple antigen detection, several ELISA kits have to be used. In addition, these methods usually require a radioisotope, enzyme, fluorescence or colloidal gold-labeled antibody or antigen and may suffer from drawbacks of requiring skilled personnel, time-consuming procedures and expensive chemicals [22].

Many healthcare systems in developed countries have strategies to improve cancer care and survival rates through improvements in cancer screening, with the emphasis on early detection. Recent advances in medical research have revealed that cancers are caused by many factors and each type of cancer can be represented by multiple cancer markers. Existing methods for cancer marker detection are based on immunoassays, which detect one protein marker at a time and are unsuitable for clinical applications that require the ability to determine multiple markers in a timely and cost-effective manner. There is a significant and pressing need for new cancer diagnostic technologies for parallel detection of multiple cancer markers and especially a piezoelectric ceramic cancer diagnostic biosensor for simultaneous detection of multiple cancer markers.

The cancer diagnostic market lies in two areas. The first lies in disease management and monitoring to observe therapeutic response and signs of recurrence. The second is screening—targeting people with increased risk because of family history, occupation, or age—for cancer. A biosensor array can be used in targeting cancer screening area for fast screening of high risk groups. It can also be used to monitor the effectiveness of treatment or recurrence of cancer after therapy.

Thus there is a need for a biosensor platform for the simultaneous detection of multiple biomarkers, for instance, not only for cancer detection but also for heart disease detection and prognosis, for food safety evaluation, environmental toxin monitoring and other applications.

### SUMMARY OF THE INVENTION

The present invention relates to a biosensor for detection of a biomarker in a sample comprising a high-frequency piezoelectric ceramic resonator sensor. In particular the high-frequency piezoelectric ceramic resonator sensor is a 40-100 MHz piezoelectric ceramic resonator sensor. The present invention relates further to the measurement of resonant frequency change using a high-frequency piezoelectric ceramic resonator sensor according to the invention.

The system described herein uses thickness extensional (TE) mode piezoelectric ceramic resonator as the sensing material. The capturing molecule which is specific for the analytes is being immobilized on the sensor surface for capturing the analyte and detection of mass change directly through resonance frequency change. For high sensitivity sample detection, high frequency of the piezoelectric material is required, this is possible as piezoelectric ceramic resonator at high frequency could be manufactured easily for sample detection whereas, quartz crystals cannot. Thus the system setup is sensitive for detecting low analyte level that provides valuable information in clinical application range. Besides, TE mode ceramic resonators (poled in thickness direction) are more effective for very flexible structures when compared to thickness-shear mode ceramic resonators (poled in the longitudinal direction) that are more effective for stiffer structures, thus suitable for array type biosensor development. In addition, TE mode provides uniform displacement along the thickness direction thus providing uniform measurements. Thus the sensing material or sensing mechanism is different from that described in other publications using quartz, cantilever or acoustic wave detection mechanism.

The system neither uses surface acoustic wave sensor nor a hydrogel located on the surface of the sensor wherein a molecular recognition component is immobilized as disclosed in EP 1 804 059 A2. The detection of the sample according to the invention is based on the direct resonance frequency change of the piezoelectric ceramic resonator sensor due to sample application. The detection of the analyte is directly due to the interaction of capturing molecule on the surface of sensor and the analyte present in the sample that lead to mass change which is reflected by the resonance frequency change, not due to the interaction that affect the propagation of wave through hydrogel as described in EP 1 804 059.

The sample detection sensor units could be arranged in single or array format. In one embodiment, multiple biomarkers can be simultaneously monitored by driving different resonators with different frequencies. Typically the sensor is mounted on a printed circuit board through standard soldering method and is connected to an oscillator. In one embodiment, the resonator sensor includes two piezoelectric resonators aligned in series and separated by a gap, one of the resonators being the reference resonator and the other resonator having a coating layer on the electrode surface with a capture molecule for the biomarker being immobilized thereupon.

The ceramic material of the resonator is preferably a synthetic polycrystalline ferroelectric ceramic, preferably lead titanate zirconate (PZT). The gold electrodes are constructed on ceramic material directly through sputtering method and there is no need for SiO₂ deposition (as described in EP 1 804 059) on top of the gold electrode before capturing molecules attachment for sample analysis. The capturing molecule is immobilized directly on the sensor gold electrode surface through linker group which is used for sample detection to measure the mass change directly.

In one embodiment, the biosensor includes the piezoelectric ceramic biosensor with capture molecules for biomarkers and a sample loading cartridge. In a further embodiment, the biosensor additionally includes means for recording the binding interaction between the capture molecule and the biomarker; and means for data analysis and correlation of the measured frequency to biomarker amount and its differential difference to that of the normal amount in the sample. The analyzed samples include for instance blood, serum, plasma, urine or any non-organic solution depending on the biomarkers to be detected. The samples used may be for disease diagnosis, or other applications such as food analysis, environmental pollution analysis and biodefense applications. The present invention addresses several important technical and practical issues: the technical feasibility of using piezoelectric ceramic resonators as a central component of biosensor devices, and the technical feasibility of applying the ceramic-based biosensor devices in molecular diagnostics.

The present invention has several important applications including disease diagnosis and in particular, cancer in humans. The subject technique offers simultaneous detection of multiple cancer markers with simple, fast and high throughput operation, and reduced cost. The biosensor platform established can also be used for development of a series of products for other applications including but not limited to cardiovascular disease diagnostics; autoimmune disease diagnostics, coagulation disorder diagnostics, food analysis etc.

More particular, tumor biomarkers can be used to detect and analyze the tendency of cancer development using the biosensor that includes a piezoelectric ceramic resonator. Sometimes the tumor biomarker molecules will be referred to herein as a tumor "biomarker" or a similar term to denote a physiological origin for these molecules. Preferred use of the invention generally involves detection and quantitation of biomarkers that have been found to be indicative of the presence or tendency of disease development.

For example, the presence or the tendency of cancer development can be determined with high sensitivity and selectivity by detecting and quantifying the tumor biomarkers. The preferred use of the subject invention is via a bench top biosensor that is connected to a computation system, thereby facilitating simple and convenient test of samples by technical staff in a hospital or doctors at clinics. The biosensor and biosensor array described herein is well suit for the simultaneous detection of multiple cancer biomarkers and development of biosensor for other disease diagnostics such as cardiovascular diseases, autoimmune diseases, coagulation disorder, food safety analysis etc. With the use of the subject system, the opportunities for early medical intervention are increased.

In particular, the technology platform consists of a piezoelectric ceramic biosensor with capture molecules for biomarkers, a sample loading cartridge, a computation system for signal output and analysis for monitoring biomarker levels in samples such as blood, serum, plasma, and urine. The biosensor array can be used for simultaneous detection of multiple biomarkers including but not limited to detection of multiple cancer markers for screening and early diagnosis of the most common types of cancers; detection of multiple heart disease markers for early diagnosis and prognosis of heart disease; detection of multiple food contaminants for improvement of food safety; detection of multiple environmental toxins for monitoring environmental pollution and detection of multiple bio-warfare agents for military and civil defence applications.

In a preferred embodiment, the invention provides a technology platform with biosensor including a surface substrate for immobilization of a first molecule that specifically binds a second molecule; a sample application cartridge containing sample pad or microfluidic channels for sample loading; a means of detecting binding interaction between the capturing molecule and the biomarker in the sample in terms of a frequency shift; and means for evaluating and correlating the binding of the molecules to the tendency of disease development.

More particularly, the present invention also relates to a method for detection and evaluation of the amount of one or more biomarkers in a sample using the subject biosensor or the biosensor array of the invention, wherein the resonance frequency change before and after sample application is determined and correlated to the mass of the biomarker being captured by the capture molecule on the ceramic resonator surface. Specifically, the subject method includes applying an oscillating electric field across the biosensor, measuring at least one resonance frequency of the biosensor as the pre-sample application resonance frequency, providing the sample through a sample loading cartridge and incubating for interaction of capture molecule and the biomarker, applying an oscillating electric field across the biosensor, measuring at least one resonance frequency of the biosensor as the post-sample application resonance frequency, correlating the frequency change before and after sample application to the surface mass and/or surface density according to default standard range, thereby detecting and analyzing the output of one or more biomarkers. In a preferred embodiment, a computational system correlates surface mass and/or surface density with the amount of biomarker bound by the capture molecule.

In another preferred embodiment, the biosensor is sensitive to minute differences in measurement of mass. Preferably, the biosensor comprises a piezoelectric ceramic resonator as a surface substrate for immobilizing a first molecule. The principle components of the biosensor system include a piezoelectric ceramic resonator sensor, an oscillator and a control circuit. In the piezoelectric ceramic resonator sensor there are typically two carefully matched ceramic resonators coupled in series and separated by a small gap. Carefully matched ceramic resonators are also aligned in series and separated by a small gap and displayed in a square format in multiple biomarker detection. Both ceramic resonators in the case of single marker detection and all the ceramic resonators in the case of multiple biomarker detection can be exposed to the sample whereby one of the ceramic resonators is used as reference resonator for environmental control. The difference in frequency of pre and post sample application and with the reference resonator frequency change is the frequency change due to biomarker interaction which is a very sensitive indication of the mass being deposed on the ceramic resonator surface. The frequency change is proportional to the mass of the biomolecule being captured by the capture molecule on the sensing area. This frequency change is electronically recorded and transferred to a computer for data analysis.

In another preferred embodiment, the biosensor platform includes an integrated circuit for sensor control, signal recording and conditioning, temperature monitoring and microcontroller for data acquisition and data formatting.

In another preferred embodiment, the ceramic resonator biosensor includes an integrated circuit for measurement of biosensor's resonant frequency over a narrow range of impedance.

Other examples of ceramic resonator biosensors include but are not limited to ceramic resonator biosensors that are used to measure the mass of a substantial drop of target molecule contained in a sample solution. The measurement is carried out in the gas phase. Thus there is no need for the correction of viscous damping losses.

In another preferred embodiment, ceramic resonator biosensors include an integrated circuit to measure, in addition to determining mass, the molecular species of the material deposited on the ceramic resonator biosensor's reaction centre.

In one aspect, the amount of each biomarker is measured in the sample as the resonant frequency change before and after sample loading. The mass change represented by the resonant frequency change is correlated to the default standard mass range. An increase in the frequency change indicates the amount of biomarker presented in the sample and gives an indication relative to the development or tendency of development of cancer or other type of diseases as compared to clinically relevant data. Monitoring which biomarkers are detected and at what level provide specific information on which type and the tendency for the development of a particular disease that could provide a chance for early treatment.

In another aspect, preferably a single biomarker is used in combination with one non-specific marker to the target molecule for monitoring the development or tendency of development of a particular cancer or other diseases. Preferably, multiple biomarkers are used in combination with one non-specific marker to the target molecules in the sample for simultaneously monitoring the development or tendency of development of cancers or other diseases.

In another preferred embodiment, a stable sensor-oscillating system is included for sensitive detection of biomolecule interaction on the surface of the ceramic resonator.

In another preferred method, data is generated on immobilized samples on a ceramic resonator surface, after which changes in mass are detected. Preferably, the ceramic resonator biosensor includes an integrated circuit to measure, in addition to determining mass, the molecular species of the material deposited on the ceramic resonator biosensor's reaction center. The data are transformed into computer readable form; and executing an algorithm that classifies the data according to user input parameters for detecting signals representing the biomarker level contained in a sample indicating the development or the tendency of development of a particular disease.

In another preferred embodiment, the presence of certain biomarkers is indicative of the development or tendency of development of different cancer types. For example, detection of one or more tumor markers at a certain level is indicative of the development or tendency of development of cancer.

Preferred methods for detection and diagnosis of cancer include detecting at least one or more biomarkers in a sample, and correlating the detection of one or more biomarkers with the development or tendency of development of cancer, wherein one or more protein markers are selected from tumor markers such as Alpha-Fetoprotein (AFP); Cancer Antigen (CA): CA 19-9; CA 27.29; CA 125; Carcinoembryonic Antigen (CEA); beta subunit of human chronic gonadotropin (β-hCG); and Prostate-Specific Antigen (PSA).

According to the invention, the sample is preferably a fluid, for example, blood, serum, plasma, urine, and the immobilized capture molecule is a DNA probe, an RNA probe, a carbohydrate, a protein, an antibody, a fragment, a variant or derivative thereof or any other molecule that specifically binds at least one or more of the biomarkers, preferably, but not limited, tumor markers.

In another preferred embodiment, the sample is any non-organic solution containing the target molecule, for example, solution containing food components, a polluted water sample in which the agent can be an antibody, DNA, or another molecule that specifically binds at least one or more of the markers.

In another preferred embodiment, the sample is loaded through a sample loading cartridge, wherein a sample loading pad or microfluidic channel is embedded.

When multiple biosensors are used in a microsensor array, each resonator is driven with a slightly different frequency.

The frequency range of the ceramic resonators is the same. However, to prevent interference between the resonators when they are arranged in array format, the frequency for the adjacent resonators would have frequency difference of 1 MHz (e.g. adjacent resonators would have frequencies: resonator 1 with frequency of 40MHz and resonator 2 with frequency of 41 MHz or 39MHz respectively).

When measuring interaction of different antigen-antibody, it is the frequency difference pre- and post-sample addition that matters not the absolute frequency recorded. The frequency difference for different marker could be the same or different which depends on the presence and amount of biomarker in the sample solution. The specificity of the detection depends on the specific antibody immobilized on different ceramic resonators in the array format, not on the frequency difference. The frequency difference for pre- and post-sample addition of each marker would be compared to standard in which the frequency change was predetermined by using standard samples.

If one is using microfluidics, using microfluidic flow for sample application, the measurement can be in the following mode:
- Dry condition
   ■ Using microfluidic flow for sample addition, under the dry condition, the frequency is not measured continuously, rather two spot frequencies were recorded, one before the sample addition and the other after the sample addition. Thus the pre-sample addition frequency is measured and recorded. Then sample would be added using microfluidic setup and washed and dried before measure the frequency again. The frequency difference of the two frequencies recorded would be compared to standard frequency for each of the markers. A spigot could be introduced to control the flow. But if there is no spigot, the capillary action is good enough to drive the flow.
- Wet condition
   ■ Using microfluidic flow for sample addition, under the wet condition, the frequency could be measured continuously or two spot frequencies could be recorded.
   ■ Continuous sample addition can be achieved through microfluidic setup whereby real time frequency change can be recorded. The flow rate and volume would be recorded and the frequency change can be correlated to the predetermined frequency change of the standard of each marker measured under the same condition. By using such set up, the binding kinetic of the biomarkers can be evaluated.
   ■ Two spot frequencies measurement could be determined by sample application using microfluidic setup. The frequency before sample application would be recorded. A spigot would be used to stop the flow after fixed volume sample introduction. Then frequency after sample addition would be recorded when equilibration has been reached. The frequency change can be correlated to the predetermined frequency change of the standard of each marker measured under the same condition.

Note that the piezoelectric ceramic uses PZT. The PZT had a formula of Pbα-aMea[(MII1/3MV(2 + b)/3)zTixZr1 - x - z]O3, wherein Me represents a metal element; MII represents an acceptor element of a divalent metal element; MV represents a donor element of a pentavalent metal element; and z, b, x, α and a satisfy 0.05 ≤ z ≤ 0.40, 0 < bz/3 ≤ 0.035, 0.345 ≤ x ≤ 0.480, 0.965 ≤ α ≤ 1.020 and 0 ≤ a ≤ 0.05. Note the ceramic resonator is in the high frequency range that is 40MHz -100MHz. The property of the high frequency resonator used is very different from the low-frequency devices mentioned above.

Ceramics are available at lower costs than quartz, which is a very important consideration for medical diagnostic applications. Versatility is another key feature of those materials, as compositions can be selected and modified to achieve a desirable combination of properties. Besides, miniaturization of the ceramic material is possible which make it suitable for manufacturing of protein microarrays. Thus, only piezoelectric ceramic resonators can be used for biosensor platform development in different application as indicated.

Other aspects of the invention are described *infra*

### BRIEF DESCRIPTION OF THE DRAWINGS

These and other features of the subject invention will be better understood in connection with the Detailed Description, in conjunction with the Drawings, of which:
Figure 1 shows a schematic representation of the biosensor concept for detection of biomarkers;
Figure 2 shows a schematic representation of the biosensor concept of assembled biosensor array;
Figure 3 shows a schematic diagram for the development of piezoelectric ceramic biosensor;
Figure 4 shows a schematic diagram of the piezoelectric ceramic biosensor platform;
Figure 5 shows a schematic representation of immobilization of capturing molecule and target molecule detection;
Figure 6 shows a representation of individual cancer marker biosensor device;
Figure 7 shows a representation of microarray detection unit for simultaneous detection of 16 biomarkers. Each ceramic resonator chip on the PCB board represents an individual biosensor unit as shown in Figure 6. The ceramic resonator is connected to the oscillating circuit underneath the ceramic resonator through slot;
Figure 8 shows a schematic representation of the sample application system using sample pad for biomarker detection;
Figure 9 shows a schematic representation of the cartridge for sample introduction using lateral flow;
Figure 10 shows a schematic representation of the sample application system for microarray biosensor detection units using sample pad for biomarker detection; 10A: design and set up of the sample application system; 10B: Indication of flow direction after sample loading;
Figure 11 shows a schematic representation of the direct sample application system
Figure 12 shows a schematic representation of the sample application system for microarray biosensor detection units for direct sample application;
Figure 13 shows a schematic representation of the sample application system using microfluidic channels for biomarkers detection.

### DETAILED DESCRIPTION OF THE INVENTION

A novel biosensor was developed using piezoelectric ceramic resonators as the core component of the biosensor, and a biosensor array was developed for simultaneous detection of multiple biomarkers e.g. simultaneous detection of cancer markers for screening and early diagnosis of the most common types of cancers. The biosensor technology platform developed can be used for developing biosensor for various applications in different areas including but not limited to the diagnostic field.

The technology platform has wide application for development of different types of biosensors. The biosensor thus developed has competitive advantages in that it provides specific and sensitive detection of target molecule with simple procedure at high throughput and reduced cost.

Prior to setting forth the invention, the following definitions are provided. Unless defined otherwise, all technical and scientific terms used herein have the meaning commonly understood by a person skilled in the art to which this invention belongs. As used herein, the following terms have the meanings ascribed to them unless specified otherwise.

"Biomarker", "marker" or "target molecule' are used interchangeably herein, and in the context of the present invention refers to a protein or other molecules which is differentially present in a sample taken from patients having or having the tendency of developing cancer or other kind of diseases or food sample containing pathogens, or water sample containing pollutants as compared to sample taken from control subjects (e.g. healthy subject, clean food, or unpolluted water solution).

"Multiple" refers preferably to a group of at least about 2, preferably, at least about 16 members. The maximum number of members is unlimited, but is at least 100 members.

"Antibody" or "capturing molecule" are used interchangeably herein and refers to a polypeptide ligand substantially encoded by an immunoglobulin gene or immunoglobulin genes, or fragments thereof, which specifically binds and recognizes an epitope (e.g., an antigen). The recognized immunoglobulin genes include the kappa and lambda light chain constant region genes, the alpha, gamma, delta, epsilon and mu heavy chain constant region genes, and the myriad immunoglobulin variable region genes. Antibodies exist, e.g. as intact immunoglobulins or as a number of well characterized fragments produced by digestion with various peptidases. This includes, e.g. Fab' and F(ab)'₂ fragments. The term "antibody", as used herein, also includes antibody fragments either produced by the modification of whole antibodies or those synthesized *de novo* using recombinant DNA methodologies. It also includes polyclonal antibodies, monoclonal antibodies, chimeric antibodies, humanized antibodies, or single chain antibodies.

"Diagnosis" refers to the identification of the presence or nature of a pathological condition.

"Immunoassay" is an assay that uses an antibody to specifically bind an antigen (e.g., a marker). The immunoassay is characterized by the use of specific binding properties of a particular antibody to isolate, target, and/or quantify the antigen.

"Immobilization" refers to the fixation of capturing molecule on the surface of the substrate using different chemical or biological methods.

"The phrase "specifically binds" refers to a binding reaction that is determinative of the presence of the target molecule in a heterogeneous population of proteins and other components in the sample. Thus under designated biosensor measurement condition, the specific antibodies bind to a particular target molecule at least two times the background and do not substantially bind in a significant amount to other components present in the sample.

"Detect" refers to the identification of the presence, absence or the amount of the target molecule in the sample.

"Sample" refers to any non-organic solution containing the target molecule. A sample may be blood, plasma, serum, urine, food containing solution, water containing pollutants etc. The target molecule contained in the sample may be polypeptides, peptides, proteins, antibodies, cells, chemicals etc.

"Default standard" refers to the standard curve representing the frequency change at each concentration of the antigen using standard antigens for testing.

"Biosensor" refers to a small, portable, analytical device based on the combination of capturing molecules with an appropriate transducer and which detects chemical or biological materials selectively and with high sensitivity.

The phrase "single unit" refers to the biosensor setup that is used for the detection and analysis of a single biomarker.

"Microarray" refers to the biosensor setup that incorporates several single unit ceramic resonators together for the simultaneous detection of multiple biomarkers.

The phrase "Lateral flow" refers to the sample application technique utilizing sample pad whereby the sample containing the target molecule is loaded onto the sample pad and capillary action would drive the solution flow from the sample pad to the direction of absorption pad.

"Microfluidic" refers to the sample loading technique utilizing small channels fabricated on a minute area using photoresistant technology.

The phrase "development or tendency of development" refers to the status of the patient for the likelihood of developing certain disease based on the result obtained from the biosensor.

The phrase "high throughput" refers to the large number of sample that can be processed in a given unit of time.

### Biosensor concepts for biomarker detection

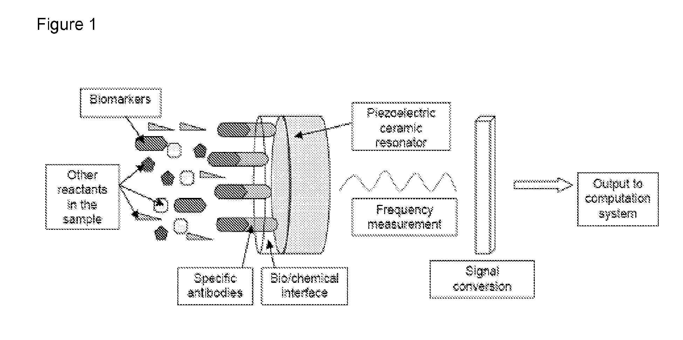

Biosensor technology provides a unique opportunity to develop diagnostic products for detection of various biomarkers. The biosensor concept includes a sensing interface comprising the specific antibody against the selected biomarker e.g. cancer markers (the antigen), and a transducer (such as ceramic resonator) that can convert the antibody-antigen binding event into an electrical signal is illustrated in Figure 1.

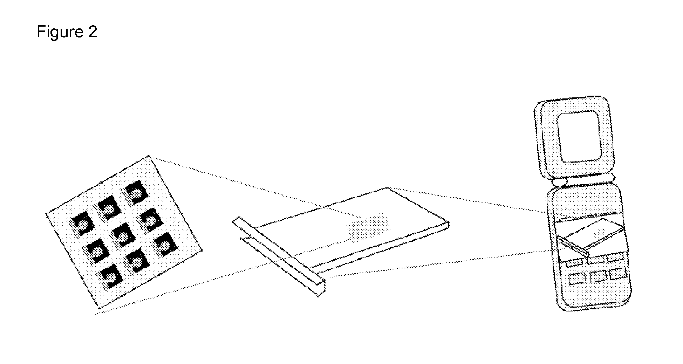

Individual biosensor unit for detecting a single biomarker can be developed first and assembled together in an array-format to produce a biosensor array that is capable of simultaneous detecting a panel of biomarkers and processed by a portable device e.g. mobile phone is illustrated in Figure 2.

Array-based immunoassays involving effective screening of antigen-antibody interactions using smaller amounts of samples can play important role in screening biomarkers with reduced cost. Results to date show that the microarray test format provides equivalent performance to ELISA, indicating that multi-parameter test can be incorporated for routine use in clinical laboratories. It also offers a significant advantage in convenience, throughput and cost when compared to traditional test formats.

By using a biosensor array as the diagnostic tool, multiple biomarkers can be detected at the same time. Miniaturization also enables the integration of multiple arrays for screening multiple patient samples. Biosensor array will have the advantages of low sample consumption with improved quantitative accuracy, sensitivity and speed.

### Piezoelectric ceramic biosensor: technical considerations

Technological change in the biosensor area has been driven in part by the successful development of home-use glucose biosensors. For the piezoelectric ceramic biosensor products, the performance characteristics and design requirements related to the use of biosensors in diagnostic test systems, and the functional requirements and processing technologies needed to bring this new technology into the diagnostic marketplace are outlined below.

### (1) Inherent Performance Characteristics

The biosensors should offer performance characteristics that would make them well-suited for applications in critical care, point-of-care, bedside, field-use and home-use test systems. The ceramic resonators are inherently smaller, more responsive, and more versatile than quartz crystals. When coupled with biomolecules, they can be formed into biosensors with high sensitivity and specificity. And by adapting manufacturing processes from the semiconductor industry, they can be produced at unit costs low enough to make their use cost-effective. The key characteristics that make the biosensors attractive are the following:
**Small Size.** To achieve the portability necessary for developing true handheld, bedside, field and home-use testing systems, miniaturization is a must. In some cases, personal convenience calls for a handheld, battery-powered unit. In others, the fact that handheld units can easily be dropped or misplaced is considered a disadvantage, making a bench top unit more desirable. The small size of ceramic sensors is an advantage in many such applications, offering a platform that can be readily adapted to a variety of formats.
**Time-to-Result.** Reductions in the size of test instruments may increase the number of settings where they can be used, but time-to-result is also a key criterion for measuring the acceptability of any such test system. Since the biosensors require smaller sample volumes than traditional analyzers, they can potentially have a faster reaction time. However, the reaction time of a sensor is generally determined by the speed at which the sample diffuses, and the speed of the molecular interactions between the analyte and the sensor's recognition element.
**Versatility.** The biosensor technology should offer a universal platform for developing other diagnostic systems for many analytes e.g. clinical, food, environmental etc. Like their larger clinical laboratory counterparts, these biosensors will inevitably compete to some degree on the basis of their menu of available tests. Using these technologies could enable manufacturers to expand their test menus. The biosensor can be applied for a wide variety of analytes, including chemistries, immunoassays, and nucleic acid assays.
**Sensitivity and Specificity.** The biosensors using high-sensitivity (high-frequency) piezoelectric transducers and high-specificity immunoassays to achieve the sensitivity and specificity required in diagnostic test systems. The biosensor should reliably detect a wide range of biomarkers with sensitivity and specificity suitable for clinical diagnostic testing e.g. cancer diagnostic, food contaminant detection, environmental pollutant detection etc.
**Ease of Manufacturing.** It is expected to make possible the production of low-cost biosensors in large quantities using the MEMS processing techniques developed by the semiconductor industry for the production of the biosensors. One stumbling block may be the incompatibility of such techniques with the processing requirements of unstable biological compounds. However, this difficulty can be overcome with the recently developed bio-dotting (microarraying) liquid dispensing techniques, such that the promise of low-cost, easily manufactured biosensors can be fulfilled.
**Cost-Effectiveness.** The actual cost of the ceramic sensor components is probably of less importance than the cost of high-quality antibodies. Nevertheless, the well established immunoassay industry should provide great flexibility in sourcing the biological components cost-effectively. In addition, advances in the biosensor design and manufacturing should further reduce the costs associated with using them.

### (2) System Requirements

Although the biosensors may inherently possess a number of characteristics that would make them useful in different application especially diagnostic applications, FDA and other regulatory agencies worldwide will have some influence over the eventual shape of any diagnostic test system that incorporates biosensors. Users also impose performance requirements for such systems, and these requirements can vary widely according to the type of user, the settings in which the system is designed to be used, and the actual types of tests the system will run. Taken together, these additional system requirements pose a considerable challenge for the biosensors. Following are some of the key areas in which regulatory or customer requirements may be important.

**Precision and Accuracy.** Clinical laboratories are accustomed to the precision and accuracy delivered by today's highly sophisticated laboratory equipment, and they are unlikely to tolerate any reduction of these parameters. The same standards are also being applied to test systems designed for use outside the clinical laboratory, meaning that even units designed for home use will be required to perform as precisely and accurately as much larger systems. This requirement will challenge us to design the biosensor systems that can be operated reliably without the intervention of trained technicians.

**Use- and Shelf Life.** The shelf life of the biosensors can be a key factor in achieving efficiencies in manufacturing, distribution, and sales. Shelf life is even more important for biosensors designed for the over-the-counter or prescription home-use market. The challenge is to design biosensor components, particularly the biological components at the sensor interface, to maintain the integrity of minute amounts of chemical or biological components for several months, preferably at ambient temperatures. The biological interfaces must also remain stable in use.

**Ease of Use.** The biosensors diagnostic systems should be easy to use. The challenge is to reduce the operational complexity of the system, which is mainly determined by the user interface. The microarray format of the biosensors can help to reduce the complexity of the user interface through automated calibration or quality control using one or some of the arrayed units.

**Whole Blood.** Use whole blood directly eliminates the need for extensive sample preparation. For the biosensors, achieving this goal may be one of the most challenging tasks of all. This is largely because of the relatively wide spectrum of pH, ionic strength, viscosity, water content, and interfering substances that can be present in a whole blood patient sample. These factors can affect the response of the biomolecules used in the biosensor, thereby reducing the accuracy and performance of the system.

### Development of a piezoelectric biosensor

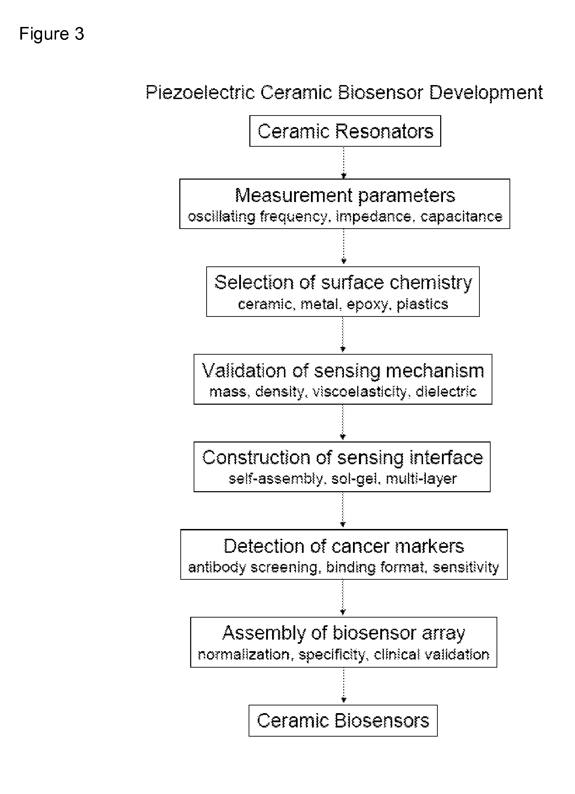

In a preferred embodiment, the specific capturing molecule e.g. antibodies (the recognition elements) against the selected biomarkers was immobilized on piezoelectric ceramic resonators for detection of biomarkers (antigens or analytes) presented in samples e.g. patient samples, food samples, water samples etc. Signals generated by the ceramic resonator including the oscillating frequency may be modulated when the resonator-antibody interface undergoes certain changes due to the binding of the antigens to the immobilized antibodies. A biosensor unit for detecting a single biomarker was developed to evaluate the feasibility of the biosensor for biomarker detection. This was followed by the development of an array-format biosensor device that is capable of simultaneous detecting a panel of biomarkers. Figure 3 shows the schematic diagram for the development of piezoelectric ceramic biosensor.

As an illustrative example, not meant to limit or construe the invention in any way, the following is provided. Biosensor for the diagnostic of cancer markers was used as an example for the biosensor technology platform development. High-throughput biosensor arrays was developed for fast patient sample screening based on the platform developed. The platform developed can also be modified to increase the number of cancer markers per array unit to increase the detection spectrum of cancer types. More tumor markers can be included when other organ specific tumor markers are identified. The platform developed can also be used for the development of biosensors for biomarkers detection in different applications such as cardiovascular disease, diabetes, food contaminants, environmental pollutants etc.

### Biosensor design

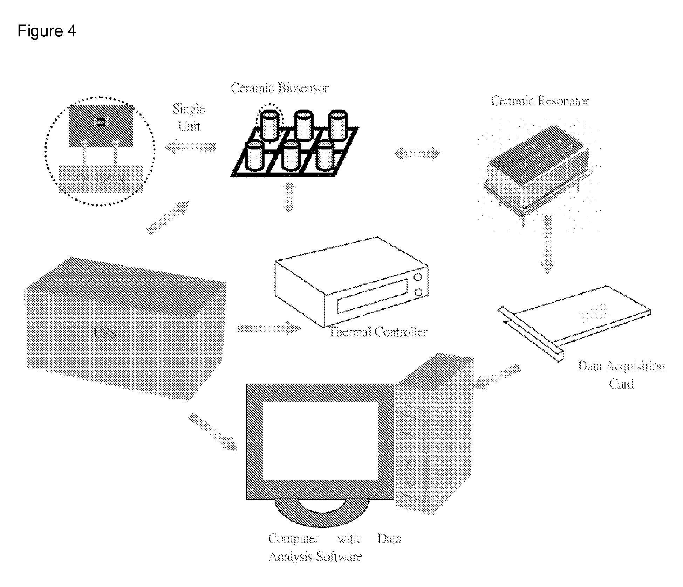

The development of the biosensor for detection of different types of biomarkers involves the utilization of the piezoelectric property of ceramic material, the specific interaction between antibodies and antigens, and the measurement of the frequency (and / or other parameters) changes due to antibody-antigen interactions. Figure 4 shows the overall design of the microarray biosensor.

The system is composed of multiple subunits in an array format, each subunit containing a different biomarker detecting component and can be addressed individually (either parallel or in series). A thermal controller may be included to control reaction temperature. The recorded frequency change during biomarker detection is sent to a data acquisition card and processed by a computer for data analysis and report generation.

### Core component: piezoelectric ceramic resonators

The sensitivity, stability, reproducibility, throughput and cost largely depend on the sensor component used. Piezoelectric ceramic resonators used should preferably possess high frequency and large surface area, small size, and compatible for immobilization of biological materials.

Ceramics are mass-sensitive transducers, their frequency is dependent on the mass of the crystal's surface as well as the mass of any layers confined to the electrode areas of the crystal. The technology of manufacturing stable, high frequency (10-100 MHz range) ceramic material is mature and these materials are readily available, whereas other piezoelectric material like quartz is not. Ceramic resonators with various frequencies (in the MHz range) and dimensions were tested first to choose the most sensitive and stable resonator with suitable surface coating for the immobilization of antibodies.

The trend for clinical diagnostic is moving towards the development of microarray format sample detection system that is suitable for high throughput screening. Thus miniaturization of the ceramic material is another issue that needs to take into consideration. The smallest dimension for commercial ceramic product is down to 0.4mm x 0.2mm at very low production cost compared to other piezoelectric material like quartz. As the material can be produced in small size with stability, reproducibility and high sensitivity, it is very suitable for the development of new generation of diagnostic kit with high throughput.

### Selection of biomarkers: cancer markers as an example

Cancer is the second leading cause of death worldwide and the total number of death due to cancers is 7.6 million accounting for 13% of total death worldwide. A WHO report in 2004 clearly indicated that lung cancer, stomach cancer, colon/rectum cancer, liver cancer, breast cancer and prostate cancer are the six most common types of cancer accounting for 60% of the total death caused by cancer. Thus, the cancer diagnostic biosensor will initially focus on the simultaneous screening of a panel of cancer markers related to these six types of cancers.

Cancer markers are soluble molecules (usually glycoproteins) in the blood/urine that can be detected by monoclonal antibodies. Each cancer marker has a variable profile of usefulness for screening, diagnosis and prognosis, assessing response to therapy, and monitoring for cancer recurrence.
***(1) Diagnostic functions of cancer markers:*** Screening tests require high sensitivity to detect early-stage disease. These tests also must have sufficient specificity to protect patients with false-positive results from unwarranted diagnostic evaluations. Biosensors for cancer marker will offer significant benefit for the general population. In addition, cancer markers can play a crucial role in detecting disease and assessing response to therapy in selected groups of patients. Table 1 lists a number of common cancer markers used in clinical settings.

**Table 1. Conditions Associated with Elevated Cancer Marker Levels**

| **Cancermarker** | **Normal value** | **Primary tumor(s)** | **Additional associated malignancies** | **Threshold Level** | **Sensitivity** |
|---|---|---|---|---|---|
| CA 27.29 | <38 units per mL | Breast cancer | Colon, gastric, hepatic, lung, pancreatic, ovarian, and prostate cancers | >100 units per mL | Elevated in about 33% of early-stage breast cancers and about 67% of late-stage breast cancers |
| CEA | <2.5 ng per mL in nonsmoker s | Colorectal cancer | Breast, lung, gastric, pancreatic, bladder, medullary thyroid, head and neck, cervical, and | >10 ng per mL | Elevated in less than 25% of early-stage colon cancers and 75% of late-stage colon cancers |
| | <5 ng per mL in smokers | | hepatic cancers, lymphoma, melanoma | | |
| CA 19-9 | <37 units per mL | Pancreatic cancer, biliary tract cancers | Colon, esophageal, and hepatic cancers | >1,000 units per mL | Elevated in 80% to 90% of pancreatic cancers and 60% to 70% of biliary tract cancers |
| AFP | <5.4 ng per mL | Hepatocellular carcinoma, nonseminomatous germ cell tumors | Gastric, biliary, and pancreatic cancers | >500 ng per mL | Elevated in 80% of hepatocellular carcinomasNonseminomatous germ cell tumors: see b-hCG below |
| b-hCG | <5 mIU per mL | Germ cell tumors, gestational trophoblastic disease | Rarely, gastrointestinal >30 mIU per cancers mL | | AFP or b-hCG elevated in 85% of nonseminomatous germ cell tumors; elevated in only 20% of early-stage nonseminomatous germ cell tumors |
| CA 125 | <35 units per mL | Ovarian cancer | Endometrial, fallopian >200 units per tube, breast, lung, mL esophageal, gastric, hepatic, and pancreatic cancers | | Elevated in about 85% of ovarian cancers; elevated in only 50% of early-stage ovarian cancers |
| PSA | <4 ng per mL for screening | Prostate cancer | Undetectable level after >10 ng per mL radical prostatectomy | | Elevated in more than 75 percent of organ-confined prostate cancers |
| NSE | <4 ng per mL | Lung cancer, neuroblastoma | Also detected in patients >10 ng per mL with Wilms' tumor; melanoma; and cancers of the thyroid, kidney, testicle, and pancreas. | | Elevated in more than 70 percent of small cell lung cancer. Measurement of NSE level can provide information on the disease stage and the patient's prognosis and response to treatment. |

| | | | | | |
|---|---|---|---|---|---|
| *CA = cancer antigen; CEA = carcinoembryonic antigen; AFP = alpha-fetoprotein; b-hCG = beta subunit of human chorionic gonadotropin; PSA = prostate-specific antigen.* Adopted from: G. L. Perkins, E. D. Slater, G. K. Sanders, and J. G. Prichard, "Serum Tumour Markers", American Family Physician, 2003, Vol. 68, pp 1075-1082 [23] | | | | | |

***(2) Reasons for targeted cancer markers***
**Cancer Antigen 27.29 (CA27.29):** Cancer antigen (CA) 27.29 is a monoclonal antibody to a glycoprotein (MUC1) that is present on the apical surface of normal epithelial cells. CA 27.29 is highly associated with breast cancer, although levels are elevated in several other malignancies. CA 27.29 also can be found in patients with benign disorders of the breast, liver, and kidney, and in patients with ovarian cysts. However, CA 27.29 levels higher than 100 units per mL are rare in benign conditions. Because of superior sensitivity and specificity, CA 27.29 has supplanted CA 15-3 as the preferred tumor marker in breast cancer. The CA 27.29 level is elevated in approximately one third of women with early-stage breast cancer (stage I or II) and in two thirds of women with late-stage disease (stage III or IV).
**Carcinoembryonic Antigen (CEA):** Carcinoembryonic antigen (CEA), an oncofetal glycoprotein, is overexpressed in adenocarcinoma, especially colorectal cancer. CEA elevations also occur with other malignancies. Non-neoplastic conditions associated with elevated CEA levels include cigarette smoking, peptic ulcer disease, inflammatory bowel disease, pancreatitis, hypothyroidism, biliary obstruction, and cirrhosis. Levels exceeding 10 ng per mL are rarely due to benign disease.
CEA values are elevated in approximately 50 percent of patients with tumor extension to lymph nodes and 75 percent of patients with distant metastasis. The highest values (above 100 ng per mL) occur with metastasis. The American Society of Clinical Oncology recommends monitoring CEA levels every two to three months for at least two years in patients with stage II or III disease who are surgical candidates.
**Cancer Antigen 19-9 (CA 19-9):** Elevated levels of CA 19-9, an intracellular adhesion molecule, occur primarily in patients with pancreatic and biliary tract cancers but also have been reported in patients with other malignancies. This tumor marker has a sensitivity and specificity of 80 to 90 percent for pancreatic cancer and a sensitivity of 60 to 70 percent for biliary tract cancer. The positive predictive value of levels over 1,000 units per mL is 97 percent when CA 19-9 testing is used in clinical situations that are consistent with pancreatic cancer. Furthermore, CA 19-9 levels above 1,000 units per mL predict the presence of metastatic disease.
**Alpha-Fetoprotein (AFP):** Alpha-fetoprotein (AFP) is the major protein of fetal serum but falls to an undetectable level after birth. The primary malignancies associated with AFP elevations are hepatocellular carcinoma and nonseminomatous germ cell tumors. Patients with cirrhosis or viral hepatitis may have abnormal AFP values, although usually less than 500 ng per mL. Pregnancy also is associated with elevated AFP levels, particularly if the pregnancy is complicated by a spinal cord defect or other abnormality.
AFP levels are abnormal in 80 percent of patients with hepatocellular carcinoma and exceed 1,000 ng per mL in 40 percent of patients with this cancer. Retrospective studies showed improved survival with AFP screening. In patients with a hepatic mass and risk factors for hepatocellular carcinoma, an AFP level above 500 ng per mL is often used in lieu of biopsy to diagnose hepatocellular carcinoma.
**Beta Subunit of Human Chorionic Gonadotropin (β-HCG):** The β-hCG normally is produced by the placenta. Elevated β-hCG levels most commonly are associated with pregnancy, germ cell tumors, and gestational trophoblastic disease. Both AFP and β-hCG play crucial roles in the management of patients with nonseminomatous germ cell tumors. The AFP or β-hCG level is elevated in 85 percent of patients with these tumors.
Following AFP and β-hCG levels is imperative in monitoring response to treatment in patients who have nonseminomatous germ cell tumors. Patients with AFP and β-hCG levels that do not decline as expected after treatment have a significantly worse prognosis, and changes in therapy should be considered. Because curative salvage therapy is possible, the tumor markers are followed every one to two months for one year after treatment, then quarterly for one year, and less frequently thereafter.
**Cancer Antigen 125 (CA 125):** CA 125 is a glycoprotein normally expressed in coelomic epithelium during fetal development. Elevated CA 125 values most often are associated with epithelial ovarian cancer, although levels also can be increased in other malignancies. CA 125 levels are elevated in about 85 percent of women with ovarian cancer. Annual ultrasound examination and CA 125 screening have been advocated for women. Currently, ovarian cancer is treated with maximal surgical reduction, which leaves minimal clinical or radiographic disease. After definitive treatment of ovarian cancer, CA 125 levels should be obtained every three months for two years, and with decreasing frequency thereafter. Elevated CA 125 levels during follow-up nearly always indicate ovarian cancer recurrence.
**Prostate-Specific Antigen (PSA):** PSA is a glycoprotein produced by prostatic epithelium. The PSA level can be elevated in prostate cancer, prostatitis, benign prostatic hypertrophy, and prostatic trauma. Prostate cancer screening is suggested for black men at age of 40 years and in all men with a family history of prostate cancer. In patients without established risk factors, screening could begin at age 50. If elevated PSA values are confirmed, patients should be referred for biopsy. After treatment of prostate cancer, PSA levels should be obtained every six months for five years, and then annually. In men who have undergone radical prostatectomy, any detectable PSA is significant.
**Neuron-specific enolase (NSE):** NSE is the neurol isoenzyme of the intracytoplasmic enzyme enolase, which was first found in the extract of brain tissue and was later shown to be present in neuroendocrine cells and tumors. NSE is a well known marker of lung cancer. Previous study showed that increased values of NSE were present in the cancer patients and that NSE was significantly correlated with tumour diameter and disease extent. Elevated serum concentrations of NSE have been found in over 70% of patients with small-cell lung cancer (SCLC). Measurement of NSE level in patients with lung cancer can provide information about the extent of the disease and the patient's prognosis, as well as about the patient's response to treatment.

The above eight protein cancer markers were chosen: CA25.29, AFP, CA125, CA19-9, CEA, β-HCG, NSE, and PSA. These markers cover the six types of most common cancers (lung, stomach, colon/rectum, liver, breast and prostate cancer) that account for more than 60% of the total cancer related death, and also relate to five other cancers types: neuroendocrine cancer, pancreatic cancer, digestive system organ cancer, ovary cancer and testicular cancer.

Each of the above markers is currently used either in isolation or in combination for a variety of cancers for screening, diagnosis, planning appropriate therapy, monitoring response to therapy (chemotherapy or radiotherapy), following-up care to check for recurrence or as a screening tool of those people in high-risk groups. A diagnostic technology that can detect the levels of all the above markers at the same time should offer the doctors a powerful tool in screening, diagnosis, and management of the most common cancers.

### Immobilization of antibody

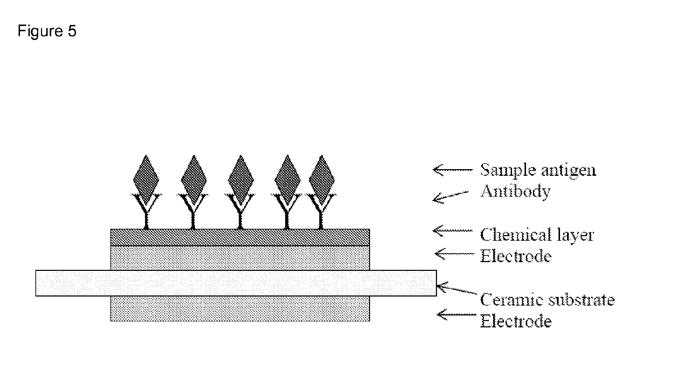

When the biomarkers or markers are selected e.g. cancer markers, their respective antibody could be immobilized on the ceramic material for detection of the antigen. Figure 5 is a schematic representation of the immobilization and antibody-antigen interaction.

Piezoelectric ceramic resonator consists of ceramic wafer of different thicknesses sandwiched between two electrodes, which provide a means of connecting the device to an external oscillator circuit that drives the ceramic crystal to oscillate at its resonant frequency. Electrodes are constructed on ceramic material directly through sputtering method. For detection to take place, the antibody has to be immobilized on the ceramic surface first. There are many different methods for combining the ceramic sensor component with the biological sensing element (antibody against cancer marker), depending on the surface chemistry and the materials and the packing of the ceramic resonators. As an example, assuming that the antibodies will be immobilized on the electrode surface (which presumably is the most sensitive areas of the resonator), different types of antibody immobilization methods were tested to determine the more suitable method for antibody immobilization and sample detection.

### (1) Self-assembly technique: Sulfo-LC-SPDP chemistry

In one preferred embodiment, antibody was immobilized on the gold electrode's surface of ceramic crystal by self-assembled technique using sulfosuccinimidyl 6-[3'-(2-pyridyldithio) propionamido] hexanoate (sulfo-LC-SPDP). The highly ordered self-assembled monolayers (SAM) ensure well-controlled surface structure and offer many advantages to the performance of the sensor. The formation of SAM is due to the strong and spontaneous adsorption between sulfur atoms of sulfo-LC-SPDP and a gold surface. Sulfo-LC-SPDP is a good thiol-cleavable heterobifunctional cross-linker, for it contains one *N*-hydroxysuccinimide residue and one pyridyl disulfide residue. The pyridyl disulfide residue of Sulfo-LC-SPDP can be used to introduce sulfhydryl groups into proteins or to conjugate an amine-containing protein to a sulfhydryl-containing protein such as IgG type of antibody. As a result, the protein can be immobilized onto the gold surface through sulfo-LC-SPDP in a single step through covalent bond.

The resulting disulfide bonds are cleavable by a reducing agent such as dithiothreitol (DTT). Thereafter, the thiolated antibody-gold complex is covalently formed on the gold electrode of the crystal.

The immobilization procedures are as follows:
- The final concentration of antibody for immobilization was determined and optimized. The monoclonal antibody was diluted with PBS (pH 7.4) to a suitable final concentration. Preferably, a concentration of 0.05 to 2.5 mg/ml is used.
- Equal volume of monoclonal antibody and cross-linker solution (20mM sulfo-LC-SPDP (water solution)) was mixed and incubated at room temperature (RT) for 1.5 h.
- To reduce the disulfide bond of the thiolated antibody, 2µl of DTT (0.1M sodium acetate buffer, 0.1 M NaCl, pH 4.5) was added and reacted for 30 min.
- The mixture solution was then spread on the entire surface of one gold electrode and dried for about 1 h at ambient temperature. Preferred volume is from 0.2 to 5µl.
- The antibody-coated resonator, thus prepared was consecutively washed with PBS and distilled water, then blown dry with a stream of nitrogen gas.

The important advantages of using SAM as a platform for linking antibody molecules are of easy formations of ordered, pinhole-free and stable monolayers, flexibility to design the head group of SAM with various functional groups in order to accomplish hydrophobic or hydrophilic surface as per the requirement, minimum amount of biomolecule needing for immobilization on SAM, reasonable stability for extended period allowing several reliable measurements.

### (2) Immobilization via protein A (PA) method

In another preferred embodiment, protein A was used as the substrate for antibody immobilization. This is a commonly used two step immobilization process using protein A (PA) as the linker for antibody immobilization. PA can be spontaneously adsorbed onto the surface of the gold electrodes by physical adsorption to form the PA-gold complex. The affinity constant is 108 L/mol and the adsorption is nonspecific but compact. On the other hand, PA can covalently combine with the Fc segment of IgG. The PA method's feature is that immobilized antibody's functional fragment (Fab) is outwards the crystal, which is advantageous to capture the antigen in sample solution.

The immobilization procedures are as follows:
- Optimized amount of protein A solution in PBS (pH 7.2) was dispersed on the entire surface of one gold electrode covering the gold electrode area only. A preferred temperature and incubation time is 4°C for 16h or RT for 2h. Preferred concentration of protein A is from 0.1 to 2 mg/ml and preferred volume is from 0.2 to 5µl.
- After drying at RT, the resonator was rinsed with distilled water to remove excessive PA solution.
- Antibody at suitable concentration was applied to the electrode surface, dried at RT, and washed subsequently with PBS and distilled water. Preferred concentration of antibody is from 0.05 to 2.5 mg/ml.
- After washing, the ceramic crystal was blown dry with a stream of nitrogen gas.

### (3) Immobilization via Sulfo-LC-SPDP-PA method

In another preferred embodiment, sulfo-LC-SPDP-PA was used as the substrate for antibody immobilization. This method combines the advantage of both the SAM and protein A.

The immobilization procedures are as follows:
- The final concentration of protein A for immobilization was determined and optimized. Protein A was diluted with PBS (pH 7.4) to a suitable final concentration. Preferably, a concentration of 0.1 to 2 mg/ml is used.
- Equal volume of protein A and cross-linker solution (20mM sulfo-LC-SPDP (water solution)) was mixed and incubated at room temperature (RT) for 1.5 h.
- To reduce the disulfide bond of the thiolated antibody, 2µl of DTT (0.1M sodium acetate buffer, 0.1 M NaCl, pH 4.5) was added and reacted for 30 min.
- The mixture solution was then spread on the entire surface of one gold electrode and the crystal was dried for about 1 h at ambient temperature. Preferred volume is from 0.2 to 5µl.
- The protein A coated resonator, thus prepared was consecutively washed with PBS and distilled water, then blown dry with a stream of nitrogen gas.
- Antibody at suitable concentration was applied to the electrode surface, dried at RT, and washed subsequently with PBS and distilled water. Preferred concentration of antibody is from 0.05 to 2.5mg/ml and preferred volume is from 0.2 to 5µl.
- After washing, the ceramic crystal was blown dry with a stream of nitrogen gas.

### (4)Nitrocellulose coated interface for antibody immobilization

In another preferred embodiment, nitrocellulose was used as the substrate for antibody immobilization. Nitrocellulose is frequently used as supporting surface for antibody immobilization and has wide application.

The immobilization procedures are as follows:
- Place suitable size of nitrocellulose (NC) membrane on the ceramic resonator gold centre.
- Dissolve the NC membrane with 100% DMSO and allow it to dry at room temperature. Preferred concentration of NC in DMSO is 1 mg/µl.
- After drying at RT, suitable amount of antibody was added onto the NC coated on the gold centre. Preferred concentration of antibody is from 0.05 to 2.5mg/ml and preferred volume is from 0.2 to 5µl.

### (5)Nafion modified interface for antibody immobilization

In another preferred embodiment, nafion was used as the substrate for antibody immobilization. Nafion is a polyanionic perfluorosulfonated ionomer that is chemically inert, nonelectroactive and hydrophilic that can act as a matrix support with fast and simple immobilization procedure. The excellent thermal stability, chemical inertness and mechanical strength improved the operational and storage stability of the immunosensors. Proteins have amphiphilic nature, and could be adsorbed to the solid surface through the control of pH.

The immobilization procedures are as follows:
- Dilute the 5% Nafion coating stock solution with buffer and spread suitable amount on the gold surface of ceramic resonator. Preferred final concentration of nafion is from 0.1 % to 1% and preferred volume is from 0.2 to 5µl.
- After drying at RT, suitable amount of antibody was applied on the Nafion-modified ceramic resonator for 30 min at RT. Preferred concentration of antibody is from 0.05-2.5mg/ml and preferred volume is from 0.2 to 5µl.
- Different buffer and pH system was tested for each biomarker to find the suitable immobilization condition. Preferred pH for the buffer is from pH 4.5-8.5, the buffer could be Phosphate, Tris, Citrate, etc with a concentration of 10-50mM.
- Different NaCl concentration in the buffer was tested. Preferred concentration of NaCl in the buffer is from 50-150mM.
- After incubation, the surface is rinsed with water, and dry at RT.
Or
- Dilute the 5% Nafion coating stock solution with PBS and mix with suitable amount of antibody at 1:1 ratio. Preferred final concentration of nafion is from 0.1 % to 1%. Preferred final concentration of antibody is from 0.05-2.5mg/ml.
- Spread suitable amount of the mixture on the gold surface of ceramic resonator and incubate at RT for 30min. Preferred volume is from 0.2 to 5µl.
- After incubation, the surface is rinsed with PBS and equilibrated with PBS for 200min, and dry at RT.

### (6) Blocking and binding capacity

In another preferred embodiment, blocking reagent was applied before the detection was performed. A solution of 3% BSA in PBS was used to incubate with antibody-coated ceramic resonator to block the non-occupied residues to prevent non-specific interactions. The blocking procedures are as follows:
- Dropping a droplet of BSA solution onto the coated surfaces for 1 h. Preferred volume is from 0.2 to 5µl.
- Wash with PBS and dry by a stream of nitrogen gas.

The frequency change of the ceramic resonators was measured after immobilization and blocking procedures respectively to ensure immobilization and blocking processes are successful. The frequency change ranging from 100Hz to 50000Hz depending on the type of chemistry used. The binding capacity of the sensor can now be evaluated.

### Individual biosensor unit and biosensor array

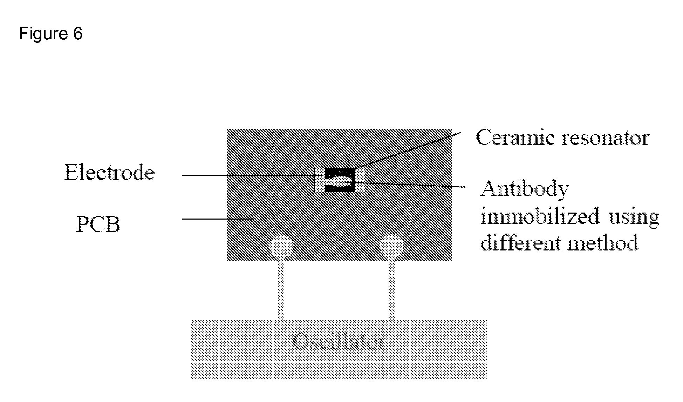

When the immobilization process is complete, biosensor detection unit for individual markers was assembled as shown in Figure 6. Sample containing antigen was added to the device and the resonance frequency change was monitored through oscillator and data acquisition. The decrease of frequency was monitored and compared to known concentration of antigen standards to evaluate the amount of antigen in the sample.

Several issues need to be addressed:
(1). Biosensor template: the sensor unit is soldered on a printed circuit board (PCB) through standard soldering method;
(2). Oscillating circuit: the circuits of various designs was tested to give the best performing stability;
(3). Liquid phase vs. gas phase: Liquid phase detection provides a real-time binding signal but may have the disadvantage of lower stability and sensitivity; Two-point (before and after) gas phase detection may be sufficient to provide the needed information;
(4). Sample handling cells: whether the sample e.g. blood will directly contact the biosensor unit or through strip by lateral flow technique where only the serum proteins are introduced to the sensor surface was tested.

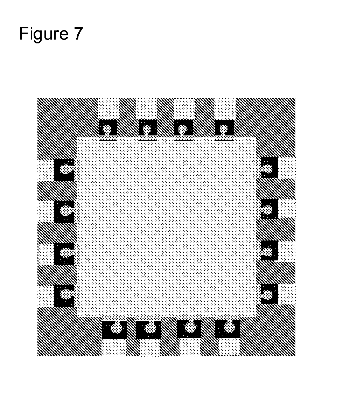

When individual biosensor device is ready, an array of biosensor units targeting at all the selected biomarkers e.g. cancer markers was assembled on a printed circuit board for parallel screening of a panel of 16 biomarkers simultaneously. The set up is shown in Figure 7.

As the dimension of ceramic resonators can be as small as 0.4mm x 0.2mm, it is possible to assemble an array of 9-16 individual biosensor units in an area of 1 cm². This offers another exciting possibility of developing a microplate (which is the standard testing format in clinical diagnosis where an array of 96 wells is manufactured on a plastic plate for parallel testing of dozens of samples) of biosensor arrays, such array of biosensor arrays will have significant implication for most of the existing microplate-based in vitro diagnostic products in clinical applications.

Standard antigen detection was performed during each step of development to ensure that the biosensor device is suitable for biomarker detection. The sample detection method and time was optimized during the testing procedure. When the microarray unit is ready, actual samples e.g. patient samples was used to evaluate the biosensor performance.

### Sample introduction and detection

In the preferred embodiment, several sample application methods were developed including sample addition using lateral flow technique, direct sample application and sample addition using microfluidic set up.

### (1) Sample introduction using lateral flow technique

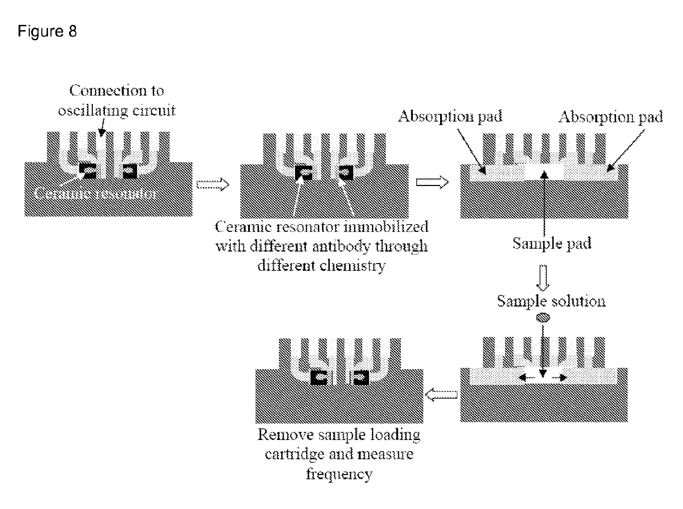

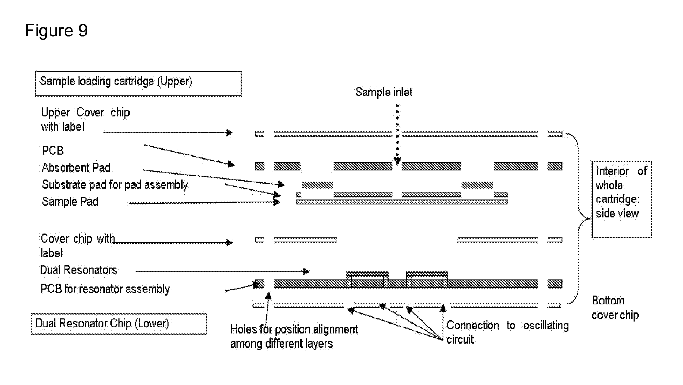

In one preferred embodiment, a sample addition strip for sample delivery using lateral flow technique was developed to press against the ceramic sensor surface; the site of interaction on which one antibody is immobilized and over which the sample e.g. serum passes during sample addition and lateral flow. Figure 8 shows the design and set up of the sample application system. The whole setup is placed in a sample loading cartridge. Figure 9 shows the cartridge containing the biosensor unit and lateral flow setup.

After coating the ceramic resonator gold centre with antibody, the biosensor is ready for sample analysis. Sensors are connected to the PCB board through standard soldering method (only two connection points are required for each resonator) and the whole PCB board is inserted into the cartridge. Only the connection points to the oscillating circuit are exposed. The sample can be applied to the biosensor by using the sample application system wherein this system consists of a cartridge containing a sample loading reservoir, the cover chip, the sample pad and absorbent pad. This sample pad will be placed against the surface of the ceramic resonator with immobilized antibody in the setup. There is only one sample inlet reservoir which is located in the middle of the ceramic resonators as indicated in the figure. After sample application in to the sample reservoir, the sample will flow due to capillary action laterally from the sample pad to the direction of the absorbent pad. External pump is not required for sample application. There is no need for the preparation of special PMMA blocks that could generate a flow channel connecting the sensors either. When the sample pass through the ceramic resonator area with immobilized capturing molecule, the antigen contained in the sample will interact with the capturing molecule specifically. Optimized time would be allowed for interaction between immobilized capturing molecule and antigen in the sample (the time would not be longer than 30 min). After sample application, PCB board containing the sensors will be removed from the cartridge and the signal is ready for detection. This signal will be compared to a pre-sample loading frequency and correlated to the default standard to generate the result.

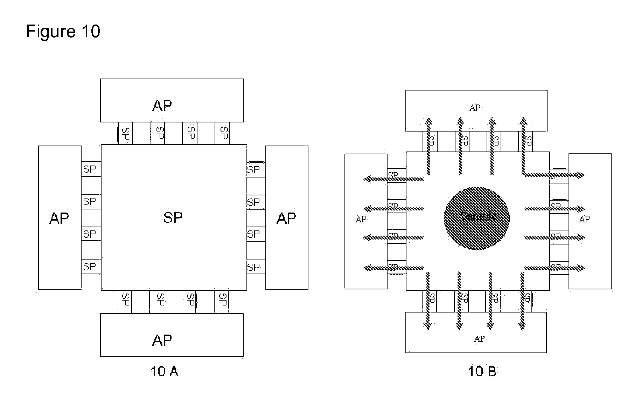

For array type biosensor, the reservoir will be located in the central area as shown in the central square areas indicated by light green color in Figure 7. Figure 10 A shows the design and set up of the sample application system whereby the sample pad (SP) and absorption pad (AP) contained in the cartridge will be placed on top of the microarray biosensor units. The cartridge setup is similar to that shown in Figure 9. When the sample is applied through the sample reservoir located in the middle, the sample containing the analyte will flow laterally in the direction as indicated in the figure 10B towards the absorption pad. When the sample pass through the ceramic resonator area with immobilized capturing molecule, the antigen contained in the sample will interact with the capturing molecule specifically. Optimized time would be allowed for interaction between immobilized capturing molecule and antigen in the sample (the time would not be longer than 30 min). After sample application, PCB board containing the sensors will be removed from the cartridge and the signal is ready for detection. This signal will be compared to a pre-sample loading frequency and correlated to the default standard to generate the result. Again, external pump is not required for sample loading.

Mass changes due to the binding antigen would lead to ceramic resonance frequency change in the device. The resonance frequency change would be recorded through the oscillator and transformed and recorded through data acquisition. The data would be processed and compared to control samples for data analysis and report generation through custom designed analysis software.

### (2) Setup for direct sample application

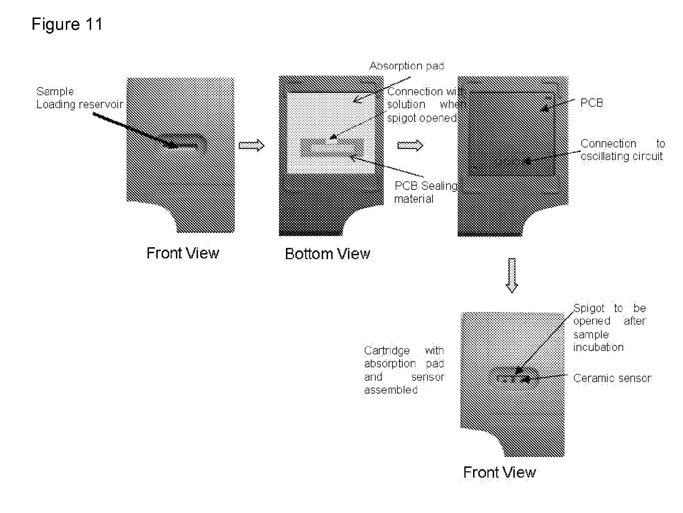

In another preferred embodiment, the sample can be administrated directly onto the sensors through the reservoir on a cartridge. The analytes contained in the sample would interact with the capturing molecule immobilized on the ceramic sensor. Figure 11 shows the design and set up of the sample application system.

After coating the ceramic resonator gold centre with antibody, the biosensor is ready for sample analysis. Sensors are connected to the PCB board through standard soldering method (only two connection points are required for each resonator) and the whole PCB board is inserted into the cartridge containing absorption pad as indicated in the figure. The sample can be applied to the biosensor by using the sample application system through the sample loading reservoir. The sample loading reservoir contains a spigot. After sample introduction, the analyte contained in the sample will interact with the capturing molecule immobilized on the sensor specifically. Optimized time would be allowed for interaction between immobilized capturing molecule and antigen in the sample (the time would not be longer than 30 min). After sample application, the spigot on the sample reservoir will be opened whereby the sample solution will be absorbed by the absorbent pad contained in the cartridge. Washing buffer can also be added through the reservoir to remove non-specific binding agents which is also absorbed by the absorbent pad and the sensor chip is ready for measurement after solution removal. This signal will be compared to a pre-sample loading frequency and correlated to the default standard to generate the result. By using this method, sample can be easily removed to prevent contamination and the whole chip is disposable which is very convenient for clinical application.

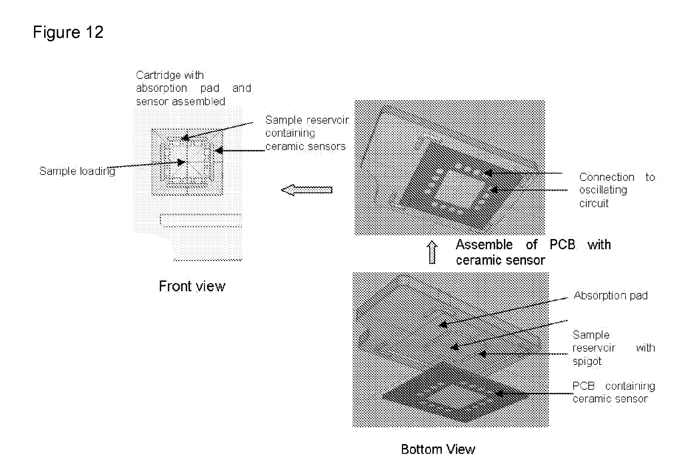

For array type biosensor, the reservoir will be located on the four sides of the microarray sensor chip as indicated in Figure 12. Figure 12 shows the design and set up of the sample application system the whole PCB containing the ceramic sensor array will be inserted into the cartridge containing absorption pad as indicated. The sample can be applied to the reservoir at the middle point of the sensor chip which is in dome shape. The applied sample will flow to the sample reservoir and the analyte contained in the sample can interact with the capturing molecule immobilized on the ceramic sensor specifically. Optimized time would be allowed for interaction between immobilized capturing molecule and antigen in the sample (the time would not be longer than 30 min). After sample application, the spigot on the sample reservoir will be opened whereby the sample solution will be absorbed by the absorbent pad contained in the cartridge. Washing buffer can also be added at the middle of the sensor chip to remove non-specific binding agents which is also absorbed by the absorbent pad and the sensor chip is ready for measurement after solution removal. This signal will be compared to a pre-sample loading frequency and correlated to the default standard to generate the result. By using this method, sample can be easily removed to prevent contamination and the whole chip is disposable which is very convenient for clinical application.

### (3) Sample introduction using microfluidic sample application setup

In another preferred embodiment, the sample can be administrated using a microfluidic sample application set up. The microfluidic-biosensor technology provides a unique opportunity to develop fractionation and diagnostic platform for online separation of sample e.g. blood cells and detection of plasma biomarkers e.g. cancer markers.

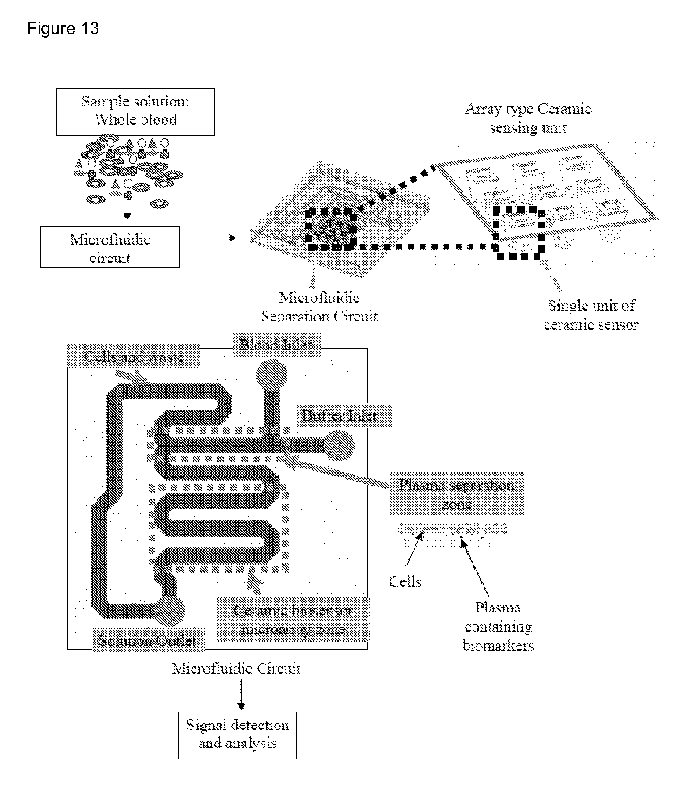

The microfluidic-biosensor includes separation unit of a microfluidic circuit for separating blood cells from plasma containing biomarkers; a sensing interface comprising the specific antibody against the selected biomarker (the antigen), and a transducer (such as ceramic resonator) that can convert the antibody-antigen binding event into an electrical signal as illustrated in Figure 13.

The sample e.g. whole blood sample containing the analytes would be fractionated by the microfluidic circuit to remove blood cells and allow plasma containing analytes to get into contact with the biosensor for analysis. The specific antibodies (the recognition elements) against the selected biomarkers e.g. cancer markers were immobilized on piezoelectric ceramic resonators for detection of plasma biomarkers (antigens or analytes) presented in patient samples. Signals generated by the ceramic resonator including the oscillating frequency may be modulated when the resonator-antibody interface undergoes certain changes due to the binding of the antigens to the immobilized antibodies.

### Fabrication of microfluidic circuit

Rapid microdevice fabrication has been demonstrated by molding PDMS against a PCB master. Channel system designs can be generated in a CAD program (CorelDRAW 12.0, Corel Corporation, UK). Improved microfluidic structure fabrication technology developed by us was used for microfluidic structure fabrication.

2400 dpi transparencies was produced by a commercial printer from the CAD files serving as photomasks and placed on top of a print circuit board (PCB, Kinsten glassepoxy single sided, Chiefskill, Taiwan). Subsequently, PCB was exposed in a standard PCB exposure unit (KVB-30 exposure unit, Chiefskill) for 80 seconds. Exposed PCB was then incubated in developing agent (Chiefskill) for 10 minutes, rinsed and wet etched with ferric chloride for 1 hour. After etching, the PCB was thoroughly rinsed before acetone removal of the remaining photoresist.

The PCB master featured with microchannels was then covered with degassed PDMS prepolymer (10 base: 1 curing agent, Sylgard 184, Dow Corning, Midland, MI) which was followed by incubation at 65°C for 1 hour. The cured PDMS replica was peeled off, trimmed and oxidized in a plasma cleaner (PDC-3XG, Harrick Scientific, Ossining, NY) for 2 min together with another thin slab PDMS placing on a piece of cleansed glass slide. The replica was then sealed against the PDMS slab to form a microdevice for sample separation in the detection platform. By this way, flow channel is created on the cartridge surface that is in connection with the PCB board containing ceramic sensors. Only one sample inlet is required and one buffer inlet and sample outlet is introduced in the setup. The sample interacts with the capturing molecule along their pathway and there is no need to use external pump. Such design not just provide the channel for sample flow, but also for separation of interfering substances before interaction of analyte with the capturing sample on the sensor surface.

### Blood sample fractionation

The plasma separation zone is a diffusion extraction device comprising microchannels with the shape illustrated in Figure 13. Buffer solution (75 µl) and a drop of sample e.g. whole blood (50 µl) can be loaded into corresponding vials sequentially. Whole blood and buffer solution are delivered into the microfluidic circuit by capillary action. A mixture of cells suspended in the whole blood sample stream enters the plasma separation zone from the top while the buffer stream (extraction stream) enters from the bottom in the fashion illustrated in Figure 13. These two streams dividing the microchannel (of plasma separation zone) in two equal halves because of their equal flow rate confined by the design. Due to the small size of the channels and the weak suction force employed for solution delivery, the flow is laminar and the streams do not mix. Under this laminar flow condition, molecular transport between blood and buffer streams occurs only by diffusion at the "plasma separation zone". Markers having a greater diffusion coefficient (smaller particles such as protein, sugars and small ions) have time to diffuse into the buffer stream (being extracted), while the larger biological cells remain in the blood sample stream and being carried away towards solution outlet vial. In this way, blood cells are separated from the interested markers, and only markers are being carried in the buffer stream and delivered towards the ceramic sensing units for detection. No matter in single or in microarray biosensor format, only one sample well will be adopted whereby the sample flows through all the sensing resonators in the flow pathway. There would be no need for external pumping and controller system for the delivery. Besides, the signals are detected by a single integrated oscillating circuit for the array resonators whereas on of the resonators would be used as reference for control purpose.

### Detection and data analysis

In preferred embodiment, the sensor in the system is made to oscillate. Upon the application of electricity, the resonances frequency can be recorded, converted to algorithm signal and exported to the computer for data analysis. The pre-sample loading resonance frequency will be recorded. The frequency after sample loading will be compared to the pre-sample loading frequency. The frequency difference between pre- and post-sample application will be analyzed by the computation system and correlated to the default standard based on relevant clinical data.

### Clinical evaluation of the biosensors

In another preferred embodiment, the working condition of the biosensor is optimized using standard antigen samples. Clinical samples (serum) from both normal and diseased cohorts were tested and compared to results obtained from standard ELISA assays and clinical diagnosis. Further improvement on the biosensor can be done based on the clinical feedbacks.

### Other biosensors developed based on the ceramic biosensor platform

The biosensor technology platform developed can easily be used for the development of other biosensors with different applications e.g. in vitro diagnostic tests.

Worldwide market size of the total *in vitro* diagnostic testing market is forecast to grow to US$45.5 billion by 2010 with point-of-care diagnostic tests accounting for 1/3 of the total worldwide *in vitro* diagnostic testing market. In one preferred embodiment, the possible biosensor is the biosensor for diagnosis of heart disease.

### Cardiovascular disease (CVD):

CVD is the leading cause of death worldwide causing 16,733,000 deaths that account for 29.3% of the death rate. Heart disease is the second leading cause of death in Japan (15.5%), Hong Kong (14.6%) and the fourth leading cause of death in China (14.4%). Around 80% of all CVD deaths worldwide took place in developing, low and middle-income countries, while these countries also accounted for 86% of the global CVD disease burden. It is estimated that by 2010, CVD will be the leading cause of death in developing countries. As well, at least 20 million people survive heart attacks and strokes every year, a significant proportion of them requiring costly clinical care, which puts a huge burden on long-term care resources. The economic cost of cardiovascular diseases and stroke in 2002 is estimated at US$329.2 billion. The true cost in human terms of suffering and lost lives is incalculable.

Cardiovascular diseases impose a heavy socioeconomic burden on different countries. In the five largest European countries, France, Germany, Italy, Spain, and the United Kingdom, a total of US$110 to US$115 billion is spent annually for the management of cardiovascular disorders. Of this total, approximately US$40 billion is spent for treating coronary heart disease, more than US$15 billion for stroke, US$12 billion for hypertensive disease, and approximately US$8 billion for congestive heart failure.

Cardiovascular is the leading therapeutic category with global sales of US$50 billion a year. With cardiovascular disease as the leading cause of morbidity and mortality worldwide, the challenge now is to develop increasingly sensitive diagnostic modalities, especially in vitro diagnostic tests that can solve the problem of early diagnosis or detection of predisposing factors. Cardiovascular diagnostic product sales, including electrocardiography, noninvasive imaging, and intravascular techniques, generated more than US$1.4 billion in 1999 for the five major countries of Europe. Key drivers are forecast to boost the market for cardiac diagnostics from US$14 billion in 2001 to US$22 billion in 2006, 12% per year. The development is emphasized on in vitro diagnostic tests, primarily because this is where the major market opportunities lie.

There are a number of well known cardiac markers that can be easily incorporated into the biosensor platform to develop fast screening biosensors for early detection of different heart diseases.

In another preferred embodiment, biosensors for autoimmune diseases and coagulation disorders detection also have large market potential.

### Autoimmune diseases:

Autoimmune diseases comprise 80 or more chronic disabling diseases that affect almost every organ system in the body e.g. nervous, gastrointestinal, endocrine system, skin, skeletal and vascular tissues. In each disease, the immune system may produce autoantibodies to the endogenous antigens, with consequent injury to the host tissues and organs. In many diseases the presence of autoantibodies antedates the disease itself.

It is estimated that the total European autoimmune disease diagnostics market will reach US$700 million in 2011 and the global autoimmune diagnostic market is worth US$2 billion. The growth is mainly due to two reasons 1) Identification of many diseases that are of autoimmune disorders in which the disease pathogenesis was not known previously e.g. Celiac disease; 2) Availability of therapeutics for many autoimmune disorders increase the need for autoimmune diagnostics for early identification and disease control.

The presences of the autoantibodies in these diseases can be detected through the developed biosensor platform for fast screening, early detection and disease monitoring and control for different autoimmune diseases.

### Coagulation disorders:

Coagulation disorders (coagulopathies) are disruptions in the body's ability to control blood clotting, an essential function of the body designed to prevent blood loss. Coagulation, or clotting, is a complex process (called the coagulation cascade) that involves 12 coagulation factors found in blood plasma and several other blood components. Each factor has a precise role in coagulation. Besides the factors, plasma carries a number of other proteins that regulate bleeding e.g. platelets. A deficiency in clotting factors or a disorder that affects platelet production or one of the many steps in the entire process can disrupt clotting and severely complicate blood loss from injury, childbirth, surgery, and specific diseases or conditions in which bleeding can occur. Coagulation disorders arise from different causes and involve different complications.

Coagulation disorders affect millions of life worldwide. Thus, every hospital needs to undertake coagulation testing resulting in more than 25,000 clinical laboratories performing coagulation testing globally. The worldwide coagulation testing market is valued at US$925 million and is growing at 10% per annum. Prothrombin time test alone account for US$125 million.

The application of biosensor platform for simultaneous detection of various coagulation disorders will enhance the efficiency of disease screening process.

In another preferred embodiment, biosensors can be used in applications include the detection of food contaminants and monitoring of pollutants.

### Food safety:

The platform technology developed can be used for developing biosensor products for application in areas other than bio-medical application e.g. food safety. With increasing environmental pollution and awareness of the health, consumers are demanding safer and healthier food.

Food analysis has a global market of US$1.4 billion which is shared by about 50 companies whereas USA and Europe companies make up 2/3 of them. Food microbiological tests has a global market of US$705 million in which the rapid testing methods account for US$145 million or 20% of the total market share.

The biosensor developed can be used for food analysis in food industry to monitor food manufacturing process to ensure food safety during production. The biosensor could also be used by end user at home to monitor food safety for both fresh and processed food e.g. biosensor for monitoring of multiple food pathogens: *Clostridium botulinum; Salmonella typhi; Salmonella paratyphi; Shigella dysenteriae; Vibrio cholerae; E. coli; Listeria monocytogenes,* etc.

While the present invention has been described in connection with the preferred embodiments of the various figures, it is to be understood that other similar embodiments may be used or modifications or additions may be made to the described embodiment for performing the same function of the present invention without deviating therefrom. Therefore, the present invention should not be limited to any single embodiment, but rather construed in breadth and scope in accordance with the recitation of the appended claims.

### REFERENCES

1. Marx, K.A. Quartz crystal microbalance: a useful tool for studying thin polymer films and complex biomolecular systems at the solution-surface interface, Biomacromolecules, Vol 4(5), 1099-1120, 2003
2. Percival, C.J., Stanley, S., Galle, T.M., 2001. Molecular-imprinted, polymer-coated quartz crystal microbalances for the detection of terpenes. Anal. Chem. 73 (17), 4225-4228.
3. Wu, T.-Z., 1999. A piezoelectric biosensor as an olfactory receptor for odour detection: electronic nose. Biosens. Bioelectr. 14, 1-13.
4. Yokoyama, K., Ikebukuro, K., 1995. Highly sensitive quartz crystal immunosensors for mutisample detection of herbicides. Anal. Chim. Acta 304, 139-145
5. Guibault, G.G., Hock, B., Schmid, R., 1992. A piezoelectric immunobiosensor for atrazine in drinking water. Biosens. Bioelectr. 7, 411-419.
6. Yuan, J.-B., Tan, Y.-G., Nie, L.-H., Yao, S.-Z., 2002. Piezoelectric quartz crystal sensors based on ion-pair complexes for the determination of cinchonine in human serum and urine. Anal. Chim. Acta 454, 65-74.
7. Su, X.-D., Chew, F.-T., Li, S.F.Y., 2000. Piezoelectric quartz crystal based label-free analysis for allergy disease. Biosens. Bioelectr. 15, 629-639.
8. E.I. Du Pont De Nemours and Company, Assay method for biological target complexes on the surface of a biosensor, WO 91/05261, 18.04.1991.
9. Setter N edited, Piezoelectric materials in devices, Ceramics Laboratory, EPFL, Switzerland, 2002
10. Atonomics APS, Microsensors and method for detecting targeting analytes, WO 2002/020832, 14.03.2002.
11. Drexel University, Piezoelectric cantilever sensor, WO 2005/043126, 12.05.2005.
12. Valtion Teknillinen Tutkimuskeskus, Micromechanical sensor, sensor array and method, WO 2007/006843, 18.01.2007.
13. Atonomics APS, Surface acoustic wave sensor comprising a hydrogel, EP 1804059, 04.07.2007;
14. Lee JH, Hwang KS, Park J, Yoon KH, Yoon DS, Kim TS. Immunoassay of prostate-specific antigen (PSA) using resonant frequency shift of piezoelectric nanomechanical microcantilever. Biosens Bioelectron. 2005 Apr 15;20(10):2157-62.
15. Verissimo MIS, Mantas PQ, Senos AMR, Oliveir JABP, Gomes MTSR. Suitability of PZT ceramics for mass sensors versus widespread used quartz crystals. Sensors and Actuators B 2003; 95:25-31.
16. Eklund A, Backlund T, Lindahl OA. A resonator sensor for measurement of intraocular pressure--evaluation in an in vitro pig-eye model. Physiol Meas. 2000 Aug;21 (3):355-67.
17. The World Health Report 2005
18. China statistic 2005
19. Number of Deaths by Leading Causes of Death by Sex by Age, 2005, Hong Kong statistic 2005.
20. WHO cancer control programme, World Health Organization 2005.
21. Zakroff J, Molecular diagnostics for cancer on the rise, IVD Technology 2003.
22. Malan, P.G. (2001) in "The immunology handbook: Immunological biosensors". Wild, D. Ed. Nature Publishing group, United Kingdom.
23. G. L. Perkins, E. D. Slater, G. K. Sanders, and J. G. Prichard, "Serum Tumour Markers", American Family Physician, 2003, Vol. 68, pp 1075-1082.

## Claims

1. Biosensor for detection of a biomarker in a sample comprising a piezoelectric ceramic resonator sensor on a printed circuit board being connected to an oscillator, wherein the piezoelectric ceramic resonator sensor is a piezoelectric ceramic resonator in TE mode and wherein the piezoelectric ceramic resonator sensor is comprised of two piezoelectric ceramic resonators aligned in series to each other and separated by a gap, one of the resonators being the reference resonator and the other resonator having on the electrode surface a coating layer with a capture molecule for said biomarker being immobilized thereupon.

2. Biosensor according to claim 1 comprising a sample loading cartridge.

3. Biosensor according to claim 1 or 2 comprising means for recording the binding interaction between the capture molecule and said biomarker and means for data analysis and correlation of the measured biomarker amount to that of the normal amount in said sample.

4. Biosensor of one of claims 1 to 3, wherein the piezoelectric ceramic resonator is a high frequency resonator, preferably the frequency is between 40 and 100MHz..

5. Biosensor of one of claims 1 to 4, wherein the ceramic material of the ceramic resonator is a synthetic polycrystalline ferroelectric ceramic, preferably lead titanate zirconate (PZT).

6. Biosensor of one of claims 1 to 5, wherein the electrode surface coating layer is obtained by modification with sulfosuccinimidyl 6-[3'-(2-pyridyldithio) propionamido] hexanoate (sulfo-LC-SPDP), with protein A, with nitrocellulose or with nafion.

7. Biosensor of one of claims 1 to 6, wherein the immobilized capture molecule is a DNA probe, a RNA probe, a carbohydrate, a protein, an antibody, a fragment, a variant or derivative thereof, which preferably specifically binds to cancer biomarkers, cardiovascular disease biomarkers; autoimmune diseases biomarkers, coagulation disorder biomarkers or food analysis biomarkers.

8. Biosensor of one of claims 1 to 7, wherein the sample loading cartridge consists of a sample introduction setup, preferably a direct sample application setup, a sample addition strip or a microfluidic sample introduction setup.

9. Biosensor array for parallel detection of multiple biomarkers in various samples comprising multiple biosensors of one of claims 1 to 8 displayed in microarray format and each biosensor being designated for a different biomarker to be detected.

10. A method for detection and evaluation of the amount of one or more biomarkers in a sample using the biosensor of claims 1 to 8 or the biosensor array of claim 9, respectively, wherein the resonance frequency change before and after sample application is determined and correlated to the mass of the biomarker being captured by the capture molecule on the ceramic resonator surface.

11. The method according to claim 10 comprising applying an oscillating electric field across the biosensor, measuring at least one resonance frequency of the biosensor as the pre-sample application resonance frequency, providing the sample through sample loading cartridge and incubating for interaction of capture molecule and biomarker, applying an oscillating electric field across the biosensor measuring at least one resonance frequency of the biosensor as the post-sample application resonance frequency, correlating the frequency change before and after sample application to the surface mass and/or surface density according to default standard range, thereby detecting and analyzing the amount of one or more biomarkers.

12. The method according to claim 10 or 11, wherein a computational system correlates surface mass and/or surface density with the amount of biomarker bound by the capture molecule.
